# EUROPEAN PATENT APPLICATION

(11) **EP 4 310 188 A1**
(43) Date of publication of application: **24.01.2024**
(21) Application number: 22771464.9
(22) Date of filing: 16.03.2022
(51) Int. Cl.: C12N 15/62, A61K 35/15, C07K 19/00, A61K 39/395, A61K 47/68, A61K 48/00, A61P 21/04, A61P 43/00, C07K 14/705, C12N 1/15, C12N 5/0735, C12N 5/10, C12N 15/12, C12N 15/63

(54) **GENE CODING FOR CHIMERIC RECEPTOR FOR ANTI-ACETYLCHOLINE RECEPTOR AUTOANTIBODY**

(30) Priority: 17.03.2021 JP 2021043269
(71) Applicant: Daiichi Sankyo Company, Limited, Tokyo 103-8426 (JP)
(72) Inventor: YOSHIKIYO, Kazunori, Tokyo 103-8426 (JP); GOTO, Kazumichi, Tokyo 103-8426 (JP); TSUJIMOTO, Maki, Tokyo 103-8426 (JP); NAGASAKI, Chikako, Tokyo 103-8426 (JP); YOSHIDA, Sayaka, Tokyo 103-8426 (JP); TAGAYA, Hiroaki, Tokyo 103-8426 (JP)
(74) Representative: Marks & Clerk LLP
(86) International application number: PCT/JP2022/011820
(87) International publication number: WO 2022/196719

(57) **Abstract**

The present invention provides a chimeric polypeptide receptor in which an extracellular region comprising an antigenic region capable of being bound by an anti-human nicotinic acetylcholine receptor α1 subunit (nAChRα1) antibody, a transmembrane region, and an intracellular domain comprising an intracellular signaling domain are arranged in the presented order from the N-terminus towards the C-terminus, wherein an amino acid sequence of the antigenic region comprises the amino acid sequence as set forth in SEQ ID NO: 2 or an amino acid sequence derived from the amino acid sequence as set forth in SEQ ID NO: 2 by the substitution, deletion, insertion, and/or addition of one or several amino acids, a polynucleotide encoding the chimeric polypeptide receptor polypeptide, a cell expressing the chimeric polypeptide receptor, etc., which are useful in the treatment of myasthenia gravis.

## Description

### Technical Field

The present invention relates to a chimeric receptor polypeptide that specifically recognizes an autoantibody which binds to a nicotinic acetylcholine receptor, a polynucleotide encoding the polypeptide, a cell transfected with the polynucleotide, a medical composition for treating an autoimmune disease (particularly, myasthenia gravis) using the cell, a treatment method, a testing method, use thereof, etc.

### Background Art

It is widely known that cell therapy using T cells expressing a chimeric antigen receptor (CAR) that recognizes a cancer cell-specific antigen (CAR-T cells) specifically to destroy cancer cells expressing the antigen, is effective for cancer treatment. A similar approach has been taken in an attempt to treat an autoimmune disease by using T cells expressing a chimeric autoantibody receptor (CAAR) that specifically recognize an autoantibody (CAAR-T cells), to target pathogenic B cells expressing the autoantibody (Patent Literature 1: WO2016/070061).

The chimeric receptor is, in a broad sense, a molecule comprising three regions, i.e., an extracellular region comprising a binding region to a target molecule, a transmembrane region, and an intracellular region that transduces a stimulus into a cell. Genetically engineered T cells which express the chimeric receptor exert toxic activity against cells expressing a target molecule by binding of the chimeric receptor to the target molecule, and transduction of an activation stimulus to the T cells (Non Patent Literature 1: Stoiber et al., Cells. 2019 May 17; 8 (5): 472).

In order to obtain T cells that exert such activity, it is necessary to design a chimeric receptor that is expressed in a sufficient amount on the T cell surface (Non Patent Literature 2: Fujiwara et al., Cells 2020, 9 (5), 1182), and that has a conformation and a distribution on the cell surface appropriate for exerting toxic activity (Non Patent Literature 1: Stoiber et al., Cells. 2019 May 17; 8 (5): 472). It is also widely known to use monoclonal antibodies for the treatment of cancer and autoimmune diseases. Monoclonal antibodies are designed on the basis of native antibodies produced by cells, and methods of their expression in cells have been established. By virtue of being a secretory protein, a therapeutic antibody can be obtained by large-scale production even if it is expressed in low levels within a cell. On the other hand, chimeric receptors are completely artificially designed molecules. Since chimeric receptors are expressed within a cell membrane, factors such as the level of their expression within the cell and distribution on the cell surface are important for their potency. Thus, in theory, it appears that genetically engineered cells expressing the desired chimeric receptor may easily be prepared as long as a target molecule is determined. However, in practice, the preparation of such functional cells is associated with great difficulty and requires a great deal of trial and error, and remarkable originality and ingenuity for each individual chimeric receptor.

CAAR-T cell therapy in the treatment of pemphigus vulgaris, an autoimmune disease caused by an autoantibody against human Dsg3, has been reported (Non Patent Literature 3: Ellebrecht et al., Science 08 Jul 2016: Vol. 353, Issue 6295, pp. 179-184). According to this report, autoantibodies that recognize Dsg3 extracellular domains EC1, EC2, EC3, EC4, and EC5 were detected in the serum of patients with pemphigus vulgaris. Three types of CAARs were designed based on target antigenic regions EC1-EC3, EC1-EC4, and EC1-EC5, respectively; CAARs comprising the EC1-EC3 or EC1-EC4 domains were favorably expressed on the cell surface, and exhibited appropriate activity; on the other hand, expression of CAAR having EC1-EC5 as the antigenic region varied on the cell surface, and this CAAR also had insufficient activity.

Autoantibodies which bind to a nicotinic acetylcholine receptor α1 subunit (hereinafter, referred to as "nAChRα1" or "AChRα") are known to decrease the level of the corresponding acetylcholine receptor molecule present in the postsynaptic membrane of neuromuscular junctions, thereby inducing myasthenia gravis (MG), characterized by muscle weakness, increased susceptibility to fatigue, or respiratory problems (Non Patent Literature 4: Luo et al., J Mol Neurosci. 2010 Jan; 40 (1-2): 217-20; and Non Patent Literature 5: Luo et al., J Neurosci. 2009 Nov 4; 29 (44): 13898-908). In particular, more than half of all autoantibodies derived from patients with MG bind to a region of nAChRα1 called "main immunogenic region". The amount of autoantibodies which bind to the main immunogenic region also correlates with disease severity. Moreover, anti-main immunogenic region antibodies induce MG in rats when transferred to the rats (Makino et al., PLoS One. 2017 Oct 17; 12 (10): e0185976). One method for treating MG involves inducing remission using therapeutic steroids or other immunosuppressive drugs. However, not only is the complete remission rate as low as 150 or less when using such drugs, but these drugs also cause various adverse reactions and markedly inhibit quality of life. Therapies based on intravenous immunoglobulin (IVIg) or hemocatharsis exhibit some efficacy in steroid-resistant, intractable cases, or in severe cases, however, the effects are transient, and such approaches place a heavy burden on medical service providers and patients. An anti-complement C5 antibody (Eculizumab) has been approved in recent years for cases which are difficult to control by the above methods of treatment. However, it is associated with complications such as an increased risk of developing meningococcal infection, thereby limiting its use (Non Patent Literature 6: Sanders et al., Neurology. (2016) 87 (4), 419-425). Under these circumstances, there still remains a need to provide an effective method of treating myasthenia gravis.

### Citation List

### Patent Literature

Patent Literature 1: WO2016/070061

### Non Patent Literature

Non Patent Literature 1: Stoiber et al., Cells. 2019 May 17; 8 (5): 472
Non Patent Literature 2: Fujiwara et al, Cells 2020, 9(5), 1182
Non Patent Literature 3: Ellebrecht et al, Science 08 Jul 2016: Vol.353, Issue 6295, pp. 179-184
Non Patent Literature 4: Luo et al, J Mol Neurosci. 2010Jan; 40(1-2): 217-20
Non Patent Literature 5: Luo et al, J Neurosci. 2009 Nov4; 29(44): 13898-908.
Non Patent Literature 6: Sanders et al, Neurology. (2016) 87(4), 419-425

### Summary of Invention

### Technical Problem

It is an object of the present invention to provide a genetically engineered T cell transfected with a chimeric receptor gene, wherein the genetically engineered T cell is effective in a method of cell therapy for treating an autoimmune disease, such as myasthenia gravis, caused by pathogenic antibodies (autoantibodies) which specifically recognize a nicotinic acetylcholine receptor α subunit and cause destruction of self-tissues. The main effect of the engineered T cells is the removal of B cells and plasma cells which produce the pathogenic autoantibodies. In order to obtain T cells that exert such a function, it is necessary to design a transgene which enables the chimeric receptor to be expressed in a sufficient amount on the T cell surface, whilst retaining an appropriate conformation in the cell membrane for exerting activity.

An antigenic region of a functional chimeric receptor desirably assumes a conformation that is similar to its native counterpart because an autoantibody involved in an autoimmune disease recognizes an antigen in its native conformation. In order to express a given amount of a desired protein on cells, a chimeric receptor having an extracellular domain derived from a native protein which exists in monomeric form, can be expressed on the basis of the corresponding native gene sequence. However, if the chimeric receptor that is to be expressed has only one subunit of a multi-subunit molecule, such as an acetylcholine receptor, it is very difficult to maintain the native molecular conformation. In order to obtain a functional chimeric receptor, it is necessary to use trial and error to synthesize numerous receptors de *novo,* and test each of these to assess their function. Furthermore, in order for CAAR-T cells to exhibit the required activity, there must be a certain level of expression of chimeric receptors on the cell surface. Whilst large-scale production of secretory proteins may be achieved, it is very difficult to control the level of expression of proteins in the cell membrane.

Even if the receptor of interest is expressed in sufficient amounts on the cell surface, in order to for appropriate signal transduction into the CAAR-T cell following binding of the receptor to a target molecule and subsequent toxicity against target cells, it is necessary to retain an appropriate conformation of not only the binding domain of the receptor which specifically binds to the target molecule, but also the conformation of the whole receptor. Unlike the binding domain of CARs for targeting cancer cells which generally comprise a scFv, the structure of the binding domain of CAARs is highly variable and depends on the B cell receptor (BCR)/autoantibody that is to be targeted. Therefore, synthesis of CAARs is carried out *de novo* according to the desired target, without reference to other CAARs.

### Solution to Problem

The present inventors have conducted diligent studies to attain the object and consequently found that: an amino acid sequence corresponding to an extracellular region of nAChRα1 having an amino acid mutation introduced therein, is suitable as a binding region of a chimeric receptor, whereby the chimeric receptor is expressed in a cell membrane; and chimeric receptors comprising such a binding region in combination with various linker and intracellular domains are appropriately expressed in a cell membrane and exert cytotoxic activity. The present inventors have further pursued studies, leading to the completion of the present invention.

The present invention provides the following invention.
[1] A chimeric polypeptide receptor in which an extracellular region comprising an antigenic region capable of being bound by an anti-human nicotinic acetylcholine receptor α1 subunit (nAChRα1) antibody, a transmembrane region, and an intracellular region comprising an intracellular signaling domain are arranged in the presented order from the N-terminus towards the C-terminus, wherein the antigenic region comprises the amino acid sequence as set forth in SEQ ID NO: 2 or an amino acid sequence derived from the amino acid sequence as set forth in SEQ ID NO: 2 by the substitution, deletion, insertion, and/or addition of one or several amino acids.
[2] The polypeptide receptor according to [1], wherein the antigenic region is a region consisting of an amino acid sequence derived from the amino acid sequence as set forth in SEQ ID NO: 2 by the substitution of at least one amino acid represented by Xaa in SEQ ID NO: 3, and optionally by the deletion, insertion, and/or addition of one or several amino acids at positions other than amino acids represented by Xaa in SEQ ID NO: 3 as one or more additional mutations.
[3] The polypeptide receptor according to [2], wherein the one or more additional mutations comprises the deletion or addition of one to five N-terminal and/or C-terminal amino acids in SEQ ID NO: 2.
[4] The polypeptide receptor according to [2] or [3], wherein in the amino acid sequence of SEQ ID NO: 3, the amino acid represented by Xaa is as follows:
   Xaa8 is a hydrophobic amino acid or an acidic amino acid,
   Xaa14 is an acidic amino acid or a hydrophobic amino acid,
   Xaa70 is an acidic amino acid, an amino acid having a side chain comprising an amide group, or a hydrophobic amino acid,
   Xaa72 is a hydrophobic amino acid,
   Xaa112 is a hydrophobic amino acid,
   Xaa149 is a hydrophilic amino acid or a hydrophobic amino acid,
   Xaa155 is a hydrophobic amino acid or a hydrophilic amino acid,
   amino acids Xaa146 to Xaa148, Xaa150 to Xaa154, and Xaa156 to Xaa159 are each independently a basic amino acid or a hydrophobic amino acid, and
   Xaa192 and Xaa193 are each independently Cys or Gly, wherein the hydrophobic amino acid is Val, Ala, Leu, Ile, Gly, Trp, Tyr, Phe, Met, or Pro, the hydrophilic amino acid is Arg, Lys, Asp, Glu, Asn, Gln, or Ser, the acidic amino acid is Asp or Glu, the basic amino acid is Arg or Lys, and the amino acid having a side chain comprising an amide group is Asn or Gln.
[5] The polypeptide receptor according to [4], wherein in the amino acid sequence of SEQ ID NO: 3, the amino acid represented by Xaa is as follows:
   Xaa8 is Val or Glu,
   Xaa14 is Asp, Ala, or Gly,
   Xaa70 is Asp, Asn, or Ala,
   Xaa72 is Tyr or Phe,
   Xaa112 is Tyr or Phe,
   Xaa149 is Trp, Arg, Ala, Lys, Asp, Ser, Gly, Asn, Ile, Phe, Met, or Pro,
   Xaa155 is Trp, Arg, Ala, Lys, Asp, Ser, Gly, Asn, Ile, Phe, Met, or Pro,
   amino acids Xaa146 to Xaa148, Xaa150 to Xaa154, and Xaa156 to Xaa159 are each independently the amino acid of the corresponding site in SEQ ID NO: 2 or Lys, and Xaa192 and Xaa193 are each independently Cys or Gly.
[6] The polypeptide receptor according to [5], wherein in the amino acid sequence of SEQ ID NO: 3, Xaa149 is Trp, Lys, Arg, or Pro, and Xaa155 is Val, Ala, Gly, Lys, Arg, Pro, Met, Asp, Asn, Glu, Gln, or Ser.
[7] The polypeptide according to [6], wherein in the amino acid sequence of SEQ ID NO: 3, Xaa149 and Xaa155 are each independently Lys or Arg.
[8] The polypeptide receptor according to [1], wherein the antigenic region is any one member selected from the following group:
   antigenic region: AChRα211, AChRα211/V8E, AChRα211/W149R,
   AChRα211/W149K, AChRα211/W149P, AChRα211/V155A,
   AChRα211/V155K, AChRα211/V155M, AChRα211/V155D,
   AChRα211/V155P, AChRα211/C192G, AChRα211/C193G,
   AChRα211/C192G/C193G, AChRα211/V8E/W149R,
   AChRα211/V8E/W149K, AChRα211/V8E/W149P,
   AChRα211/V8E/V155A, AChRα211/V8E/V155K,
   AChRα211/V8E/V155M, AChRα211/V8E/V155D,
   AChRα211/V8E/V155P, AChRα211/V8E/C192G,
   AChRα211/V8E/C193G, AChRα211/V8E/C192G/C193G,
   AChRα211/W149R/V155A, AChRα211/W149R/V155K,
   AChRα211/W149R/V155M, AChRα211/W149R/V155D,
   AChRα211/W149R/V155P, AChRα211/W149R/C192G,
   AChRα211/W149R/C193G, AChRα211/W149R/C192G/C193G,
   AChRα211/W149K/V155A, AChRα211/W149K/V155K,
   AChRα211/W149K/V155M, AChRα211/W149K/V155D,
   AChRα211/W149K/V155P, AChRα211/W149K/C192G,
   AChRα211/W149K/C193G, AChRα211/W149K/C192G/C193G,
   AChRα211/W149P/V155A, AChRα211/W149P/V155K,
   AChRα211/W149P/V155M, AChRα211/W149P/V155D,
   AChRα211/W149P/V155P, AChRα211/W149P/C192G,
   AChRα211/W149P/C193G, AChRα211/W149P/C192G/C193G,
   AChRα211/V8E/W149R/V155A, AChRα211/V8E/W149R/V155K,
   AChRα211/V8E/W149R/V155M, AChRα211/V8E/W149R/V155D,
   AChRα211/V8E/W149R/V155P, AChRα211/V8E/W149R/C192G,
   AChRα211/V8E/W149R/C193G, AChRα211/V8E/W149R/C192G/C193G,
   AChRα211/V8E/W149K/V155A, AChRα211/V8E/W149K/V155K,
   AChRα211/V8E/W149K/V155M, AChRα211/V8E/W149K/V155D,
   AChRα211/V8E/W149K/V155P, AChRα211/V8E/W149K/C192G,
   AChRα211/V8E/W149K/C193G, AChRα211/V8E/W149K/C192G/C193G,
   AChRα211/V8E/W149P/V155A, AChRα211/V8E/W149P/V155K,
   AChRα211/V8E/W149P/V155M, AChRα211/V8E/W149P/V155D,
   AChRα211/V8E/W149P/V155P, AChRα211/V8E/W149P/C192G,
   AChRα211/V8E/W149P/C193G, AChRα211/V8E/W149P/C192G/C193G,
   AChRα211/W149R/V155A/C192G, AChRα211/W149R/V155K/C192G,
   AChRα211/W149R/V155M/C192G, AChRα211/W149R/V155D/C192G,
   AChRα211/W149R/V155P/C192G, AChRα211/W149K/V155A/C192G,
   AChRα211/W149K/V155K/C192G, AChRα211/W149K/V155M, /C192G,
   AChRα211/W149K/V155D/C192G, AChRα211/W149K/V155P/C192G,
   AChRα211/W149P/V155A/C192G, AChRα211/W149P/V155K/C192G,
   AChRα211/W149P/V155M, /C192G, AChRα211/W149P/V155D/C192G,
   AChRα211/W149P/V155P/C192G AChRα211/W149R/V155A/C193G,
   AChRα211/W149R/V155K/C193G, AChRα211/W149R/V155M/C193G,
   AChRα211/W149R/V155D/C193G, AChRα211/W149R/V155P/C193G,
   AChRα211/W149K/V155A/C193G, AChRα211/W149K/V155K/C193G,
   AChRα211/W149K/V155M/C193G, AChRα211/W149K/V155D/C193G,
   AChRα211/W149K/V155P/C193G, AChRα211/W149P/V155A/C193G,
   AChRα211/W149P/V155K/C193G, AChRα211/W149P/V155M/C193G,
   AChRα211/W149P/V155D/C193G, AChRα211/W149P/V155P/C193G,
   AChRα211/W149R/V155A/C192G/C193G,
   AChRα211/W149R/V155K/C192G/C193G,
   AChRα211/W149R/V155M/C192G/C193G,
   AChRα211/W149R/V155D/C192G/C193G,
   AChRα211/W149R/V155P/C192G/C193G,
   AChRα211/W149K/V155A/C192G/C193G,
   AChRα211/W149K/V155K/C192G/C193G,
   AChRα211/W149K/V155M/C192G/C193G,
   AChRα211/W149K/V155D/C192G/C193G,
   AChRα211/W149K/V155P/C192G/C193G,
   AChRα211/W149P/V155A/C192G/C193G,
   AChRα211/W149P/V155K/C192G/C193G,
   AChRα211/W149P/V155M/C192G/C193G,
   AChRα211/W149P/V155D/C192G/C193G,
   AChRα211/W149P/V155P/C192G/C193G,
   AChRα211/D14G/W149R/V155K, AChRα211/D14G/W149R/V155A,
   AChRα211/D14G/W149R/V155M, AChRα211/D14G/W149R/V155D,
   AChRα211/D14G/W149R/V155P, AChRα211/D14G/W149R/V155G,
   AChRα211/D14G/W149K/V155K, AChRα211/D14G/W149K/V155A,
   AChRα211/D14G/W149K/V155M, AChRα211/D14G/W149K/V155D,
   AChRα211/D14G/W149K/V155P, AChRα211/D14G/W149K/V155G,
   AChRα211/D14G/W149P/V155K, AChRα211/D14G/W149P/V155A,
   AChRα211/D14G/W149P/V155M, AChRα211/D14G/W149P/V155D,
   AChRα211/D14G/W149P/V155P, AChRα211/D14G/W149P/V155G,
   AChRα211/D14A/W149R/V155K, AChRα211/D14A/W149R/V155A,
   AChRα211/D14A/W149R/V155M, AChRα211/D14A/W149R/V155D,
   AChRα211/D14A/W149R/V155P, AChRα211/D14A/W149R/V155G,
   AChRα211/D14A/W149K/V155K, AChRα211/D14A/W149K/V155A,
   AChRα211/D14A/W149K/V155M, AChRα211/D14A/W149K/V155D,
   AChRα211/D14A/W149K/V155P, AChRα211/D14A/W149K/V155G,
   AChRα211/D14A/W149P/V155K, AChRα211/D14A/W149P/V155A,
   AChRα211/D14A/W149P/V155M, AChRα211/D14A/W149P/V155D,
   AChRα211/D14A/W149P/V155P, or, AChRα211/D14A/W149P/V155G
   wherein AChRα211 is the amino acid sequence as set forth in SEQ ID NO: 2,the position of each of the mentioned substitutions of AChRα211 refers to the corresponding position in the amino acid sequence represented by SEQ ID NO: 2, and in each substitution the substituting amino acid is indicated after the position.
[9] The polypeptide receptor according to any one of [1] to [8], wherein the intracellular signaling domain is an amino acid sequence derived from an intracellular domain of CD3ζ or CD3δ.
[10] The polypeptide receptor according to any one of [1] to [8], wherein the transmembrane region is an amino acid sequence derived from a transmembrane domain of any one molecule selected from nAChRα1, T cell receptor α or β chain, CD3ζ chain, CD28, CD3ε, CD45, CD4, CD5, CD8, CD9, CD16, CD22, CD33, CD37, CD64, CD80, CD86, CD134, CD137, ICOS, CD154, and GITR.
[11] The polypeptide receptor according to any one of [1] to [10], wherein the intracellular region further comprises one to three co-stimulatory domains on the N-terminus of the intracellular signaling domain.
[12] The polypeptide receptor according to [11], wherein the co-stimulatory domains are sequences derived from intracellular regions of one to three molecules selected from the group consisting of CD2, CD4, CD5, CD8α, CD8β, CD28, CD134, CD137 (4-1BB), ICOS, CD154, CITR, TNFR2, DR3, CD30, HVEM, CD27, and OX40.
[13] The polypeptide receptor according to any one of [1] to [12], further comprising a linker region consisting of 100 or less amino acids between the antigenic region and the transmembrane region.
[14] The polypeptide receptor according to [13], wherein the linker region is a portion or the whole of the amino acid sequence as set forth in SEQ ID NO: 5, 39, 40, or 41.
[15] The polypeptide receptor according to [1], wherein
   the antigenic region is AChRα211/W149R/V155K,
   AChRα211/W149R/V155A, AChRα211/W149R/V155M,
   AChRα211/W149R/V155D, AChRα211/W149R/V155P,
   AChRα211/W149R/V155G, AChRα211/W149K/V155K,
   AChRα211/W149K/V155A, AChRα211/W149K/V155M,
   AChRα211/W149K/V155D, AChRα211/W149K/V155P,
   AChRα211/W149K/V155G, AChRα211/W149P/V155K,
   AChRα211/W149P/V155A, AChRα211/W149P/V155M,
   AChRα211/W149P/V155D, AChRα211/W149P/V155P, or,
   AChRα211/W149P/V155G, AChRα211/D14G/W149R/V155K,
   AChRα211/D14G/W149R/V155A, AChRα211/D14G/W149R/V155M,
   AChRα211/D14G/W149R/V155D, AChRα211/D14G/W149R/V155P,
   AChRα211/D14G/W149R/V155G, AChRα211/D14G/W149K/V155K,
   AChRα211/D14G/W149K/V155A, AChRα211/D14G/W149K/V155M,
   AChRα211/D14G/W149K/V155D, AChRα211/D14G/W149K/V155P,
   AChRα211/D14G/W149K/V155G, AChRα211/D14G/W149P/V155K,
   AChRα211/D14G/W149P/V155A, AChRα211/D14G/W149P/V155M,
   AChRα211/D14G/W149P/V155D, AChRα211/D14G/W149P/V155P,
   AChRα211/D14G/W149P/V155G, AChRα211/D14A/W149R/V155K,
   AChRα211/D14A/W149R/V155A, AChRα211/D14A/W149R/V155M,
   AChRα211/D14A/W149R/V155D, AChRα211/D14A/W149R/V155P,
   AChRα211/D14A/W149R/V155G, AChRα211/D14A/W149K/V155K,
   AChRα211/D14A/W149K/V155A, AChRα211/D14A/W149K/V155M,
   AChRα211/D14A/W149K/V155D, AChRα211/D14A/W149K/V155P,
   AChRα211/D14A/W149K/V155G, AChRα211/D14A/W149P/V155K,
   AChRα211/D14A/W149P/V155A, AChRα211/D14A/W149P/V155M,
   AChRα211/D14A/W149P/V155D, AChRα211/D14A/W149P/V155P, or
   AChRα211/D14A/W149P/V155G;
   wherein AChRα211 is the amino acid sequence as set forth in SEQ ID NO: 2, the position of each of the mentioned substitutions of AChRα211 refers to the corresponding position in the amino acid sequence represented by SEQ ID NO: 2, and in each substitution the substituting amino acid is indicated after the position,

   the linker region is a portion or the whole of a CD8α hinge sequence (SEQ ID NO: 39), a CD28 hinge sequence (SEQ ID NO: 40), or an artificial linker sequence (SEQ ID NO: 41),
   the transmembrane region is a CD8α transmembrane domain (SEQ ID NO: 6), a CD28 transmembrane domain (SEQ ID NO: 37), or an AChRα transmembrane domain (SEQ ID NO: 38),
   the co-stimulatory domain is a 4-1BB co-stimulatory domain (SEQ ID NO: 7), a CD28 co-stimulatory domain (SEQ ID NO: 25), a GITR co-stimulatory domain (SEQ ID NO: 26), a TNFR2 co-stimulatory domain (SEQ ID NO: 27), a DR3 co-stimulatory domain (SEQ ID NO: 28), a CD30 co-stimulatory domain (SEQ ID NO: 29), an HVEM co-stimulatory domain (SEQ ID NO: 30), a CD27 co-stimulatory domain (SEQ ID NO: 31), or an OX40 co-stimulatory domain (SEQ ID NO: 32), and
   the intracellular signaling domain is a CD3ζ intracellular signaling domain (SEQ ID NO: 8).
[16] The polypeptide according to [15], wherein
   the antigenic region is AChRα211/W149R/V155K (which is an amino acid sequence of SEQ ID NO: 42 wherein Xaa14 is Asp, Xaa149 is Arg, and Xaa155 is Lys), AChRα211/W149R/V155A (which is an amino acid sequence of SEQ ID NO: 42 wherein Xaa14 is Asp, Xaa149 is Arg, and Xaa155 is Ala), AChRα211/D14G/W149R/V155K (which is an amino acid sequence of SEQ ID NO: 42 wherein Xaa14 is Gly, Xaa149 is Arg, and Xaa155 is Lys), or AChRα211/ D14G/W149R/V155A (which is an amino acid sequence of SEQ ID NO: 42 wherein Xaa14 is Gly, Xaa149 is Arg, and Xaa155 is Ala),
   the linker region is a CD8α hinge sequence (SEQ ID NO: 39), 14 consecutive amino acids from the C-terminus thereof (which are an amino acid sequence consisting of amino acids at positions 32 to 45 of SEQ ID NO: 39), an artificial linker sequence (SEQ ID NO: 41), or 4 consecutive amino acids from the C-terminus thereof (which are an amino acid sequence consisting of amino acids at positions 11 to 14 of SEQ ID NO: 41),
   the transmembrane region is a CD8α transmembrane domain (SEQ ID NO: 6),
   the co-stimulatory domain is a 4-1BB co-stimulatory domain (SEQ ID NO: 7) or an OX40 co-stimulatory domain (SEQ ID NO: 32), and
   the intracellular signaling domain is a CD3ζ intracellular signaling domain (SEQ ID NO: 8).
[17] A polypeptide consisting of an amino acid sequence, wherein the amino acid sequence of the polypeptide comprises an amino acid sequence of a signal peptide for enabling expression of the polypeptide on the cell surface, and further comprises an amino acid sequence of a chimeric receptor that exhibits 800 or higher sequence identity to an amino acid sequence of any of SEQ ID NOs: 43 to 47.
[18] The polypeptide according to [17], wherein the amino acid sequence of the chimeric receptor is an amino acid sequence of any of SEQ ID NOs: 43 to 47.
[19] A polynucleotide encoding a polypeptide according to any one of [1] to [18].
[20] A vector comprising a polynucleotide according to [19].
[21] The vector according to [20], wherein the vector is any vector selected from a plasmid vector, a retrovirus vector, and a lentivirus vector.
[22] The vector according to [20] or [21], wherein the vector is designed to enable expression of a polypeptide according to any one of [1] to [18].
[23] The vector according to [20] or [21], wherein the vector is a plasmid vector designed to enable production of a virus vector in which a polynucleotide encoding a polypeptide according to any one of [1] to [18] is enclosed.
[24] A cell transfected with a polynucleotide according to [19].
[25] The cell according to [24], wherein the cell expresses the polypeptide according to any of [1] to [18] on the cell surface.
[26] The cell according to [24] or [25], wherein the cell is any cell selected from a T cell, PBMC, an iPS cell, and an ES cell.
[27] A medicament for treating a disease associated with the production of an autoantibody which binds to an acetylcholine receptor α subunit, the medicament comprising a cell according to any one of [24] to [26] as an active ingredient.
[28] The medicament according to [27], wherein the disease is myasthenia gravis.
[29] A method for treating myasthenia gravis, comprising a step of administering a therapeutically effective amount of a cell according to any one of [24] to [26] to a subject to be treated.
[30] Use of a cell according to any one of [24] to [26] for the production of a drug for treating myasthenia gravis.

### Advantageous Effect of Invention

T cells expressing the chimeric receptor of the present invention exhibit *in vivo* cytotoxic activity against pathogenic B cells expressing an autoantibody against nAChRα1 in the body of a patient with myasthenia gravis, and as such, have a therapeutic effect on the patient with myasthenia gravis.

### Brief Description of Drawings

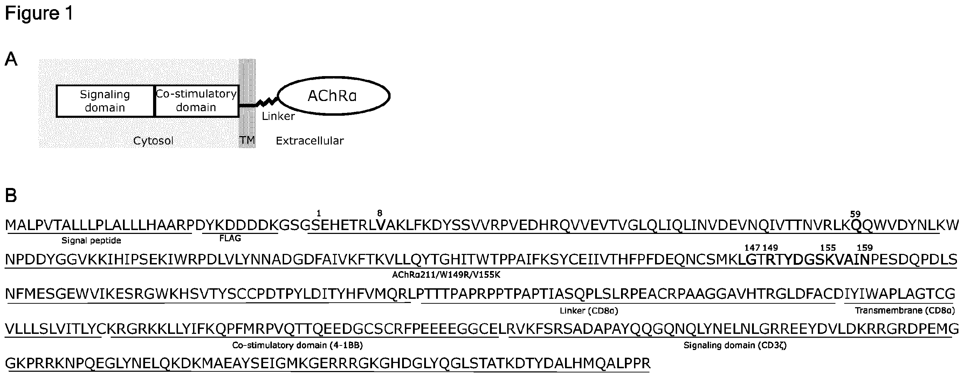

[Figure 1] Figure 1A) is a schematic diagram of AChRα-CAAR: an antigenic region (AChRα), a linker region (Linker), a transmembrane domain (TM, Transmembrane), an intracellular co-stimulatory domain, and an intracellular signal domain are linked. Figure 1B) shows the full-length amino acid sequence of an example of AChRα-CAAR: the sequence of AChRα211/W149R/V155K is shown.

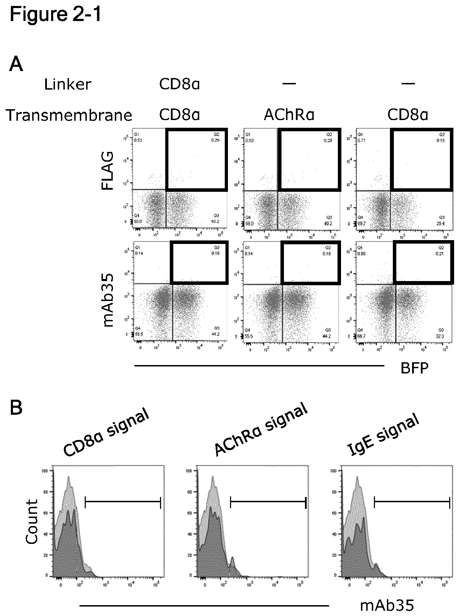

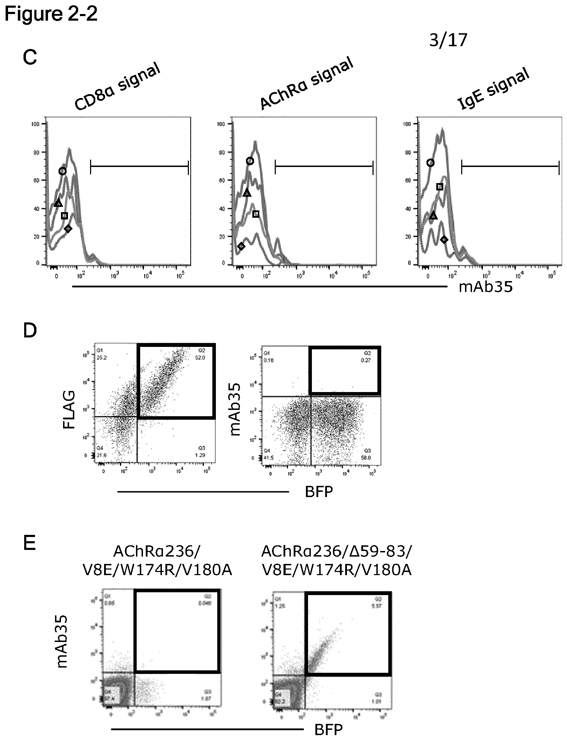

[Figure 2-1] Figure 2 shows the membrane expression of various AChRα-CAAR in human T cells as analysed by flow cytometry. In Figure 2-1(A), AChRα-CAARs were constructed and expressed by using a signal peptide (CD8α), an antigenic region (AChRα236), a co-stimulatory domain (4-1BB), and a signal domain (CD3ζ) in common, and using a linker region and a transmembrane region derived from AChRα or CD8a. The CAAR was linked to a FLAG tag peptide, and Blue Fluorescent protein (BFP) was used as a reporter for gene transfer. Figure 2-1A provides scatter plots in which the X axis depicts the expression of BFP, and the Y axis depicts the expression of the FLAG tag. Quartering lines are incorporated into the scatter plots to differentiate BFP-negative cells from BFP-positive cells, as delineated with auxiliary lines. BFP-positive/CAAR (FLAG)-positive cell fractions are delineated with thick lines. Figure 2-1(B) provides histograms showing AChRα-positive cells when AChRα-CAARs were constructed and expressed by using an antigenic region (AChRα236), a linker region (CD8α), a transmembrane domain (CD8α), a co-stimulatory domain (4-1BB), and a signal domain (CD3ζ) in common, and using a signal peptide derived from CD8α, AChRα, or IgE.
[Figure 2-2] Figure 2-2(C) provides histograms showing AChRα-positive cells when the same AChRα-CAARs as in Figure 2-1(B) were co-expressed with other AChR subunits (AChRβ, AChRδ, and AChRε). The circle depicts non-transfected T cells; the triangle depicts T cells harboring AChRα236-CAAR; the square depicts T cells harboring AChRβ, AChRδ, and AChRε; and the rhombus depicts T cells harboring AChRα236-CAAR, AChRβ, AChRδ, and AChRε. Figure 2-2(D) provides comparable scatter plots to those of Figure 2-1(A),and depicts the expression of CAAR when AChRα-CAAR was constructed and expressed by using a main immunogenic region as an antigenic region and including a signal peptide (CD8α), a linker region (CD8α), a transmembrane domain (CD8α), a co-stimulatory domain (4-1BB), and a signal domain (CD3ζ). Figure 2-2(E) provides comparable scatter plots to those of Figure 2-1(A), and depicts the expression of CAAR when AChRα-CAAR was constructed and expressed by using AChRα236/V8E/W174R/V180A (left plot) or AChRα236/Δ59-83/V8E/W174R/V180A (AChRα211/V8E/W149R/V155A; right lot) as an antigenic region and including a signal peptide (CD8α), a linker region (CD8α), a transmembrane domain (CD8α), a co-stimulatory domain (4-1BB), and a signal domain (CD3ζ).

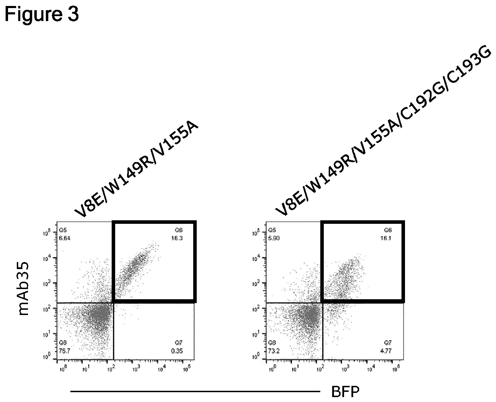

[Figure 3] Figure 3 provides scatter plots showing the expression of AChRα-CAARs having mutations corresponding to V8E, W149R, and V155A introduced in AChRα211 or AChRα236 in human T cells. The expression was evaluated on the basis of fluorescence intensity ascribable to an anti-FLAG antibody reaction. The BFP was used as a reporter for gene transfer. Quartering lines based on BFP-negative cells are incorporated as auxiliary lines. The parameters of the X axis and the Y axis, and the corresponding CAAR-T cells are shown in the plots.

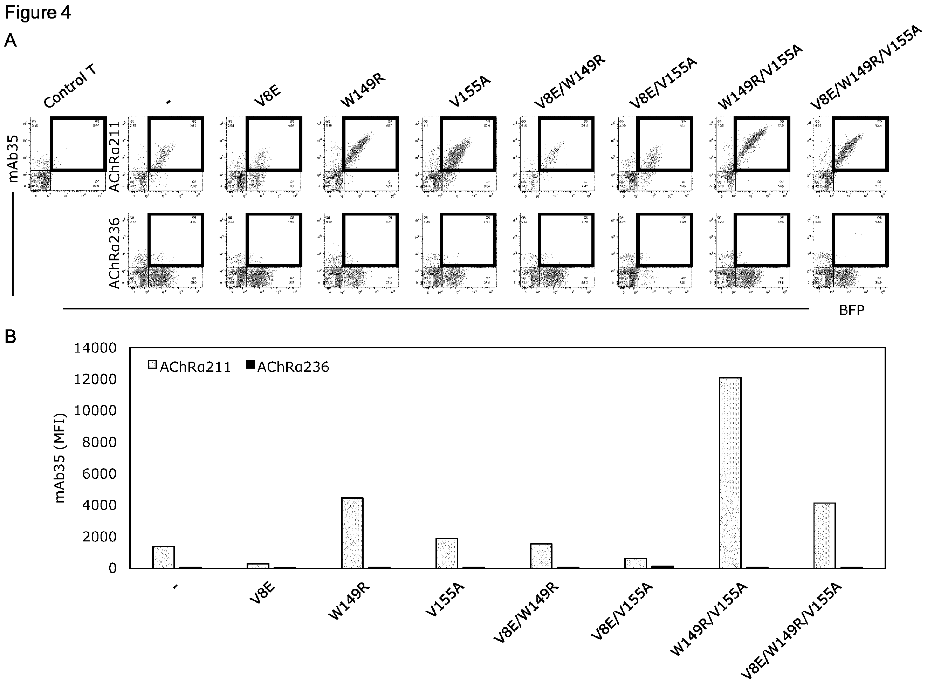

[Figure 4] The expression of AChRα-CAARs having a V8E, W149R, and/or V155A mutation introduced in AChRα211 or AChRα236 in human T cells was analyzed by flow cytometry. (A) The BFP was used as a reporter for gene transfer. Scatter plots are shown with quartering lines incorporated based on BFP-negative cells as auxiliary lines, and BFP-positive and CAAR-positive cell fractions delineated by thick lines. The X axis depicts the fluorescence intensity of BFP, and the Y axis depicts the mAb35 antibody reaction. (B) BFP-positive cells are gated from each type of transfected T cells presented in (A), and mAb35 antibody reactivity is indicated by mean fluorescent intensity (MFI).

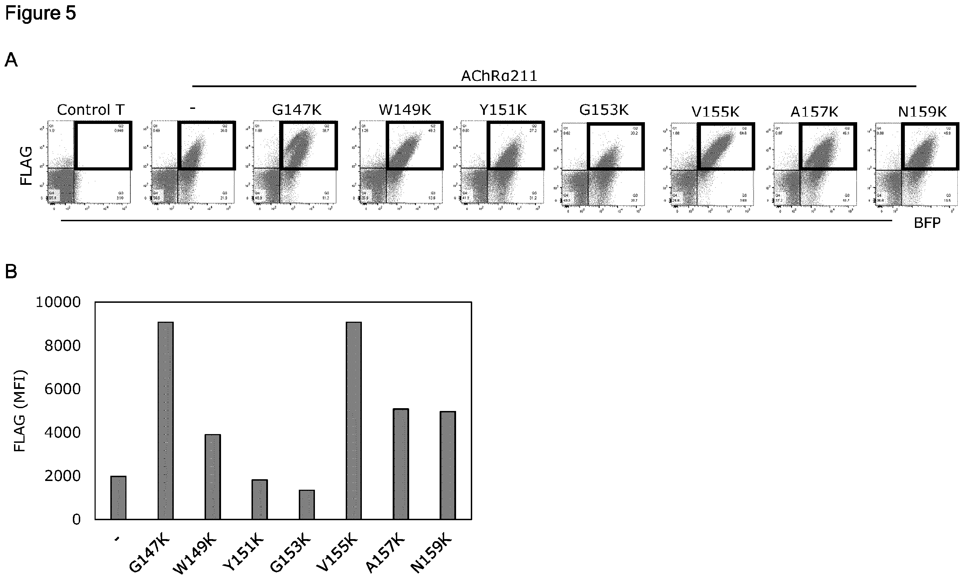

[Figure 5] The expression of AChRα-CAAR using an antigenic region having a G147K, W149K, Y151K, G153K, V155K, A157K, or N159K mutation introduced in AChRα211 in human T cells was analyzed by flow cytometry. (A) The BFP was used as a reporter for gene transfer. Scatter plots are shown incorporating quartering lines based on BFP-negative cells as auxiliary lines, and BFP-positive and CAAR-positive cell fractions are delineated by thick lines. The X axis depicts the fluorescence intensity of BFP, and the Y axis depicts the anti-FLAG antibody reaction. (B) BFP-positive cells are gated from each type of transfected T cells presented in (A), and anti-FLAG antibody reactivity is indicated by MFI.

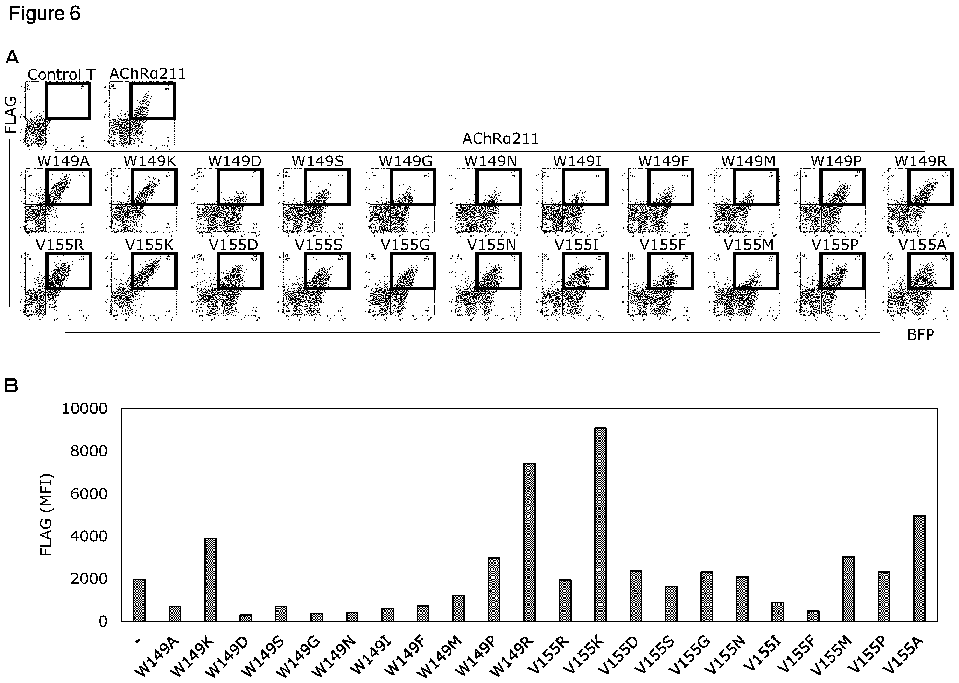

[Figure 6] The expression of AChRα-CAARs using an antigenic region having a W149A, W149K, W149D, W149S, W149G, W149N, W149I, W149F, W149M, W149P, W149R, V155R, V155K, V155D, V155S, V155G, V155N, V155I, V155F, V155M, V155P, or V155A mutation introduced in AChRα211 in human T cells was analyzed by flow cytometry. (A) The BFP was used as a reporter for gene transfer. Scatter plots are shown incorporating quartering lines based on BFP-negative cells as auxiliary lines, and BFP-positive and CAAR-positive cell fractions delineated by thick lines. The X axis depicts the fluorescence intensity of BFP, and the Y axis depicts the anti-FLAG antibody reaction. (B) BFP-positive cells are gated from each type of transfected T cells presented in (A), and anti-FLAG antibody reactivity is indicated by MFI.

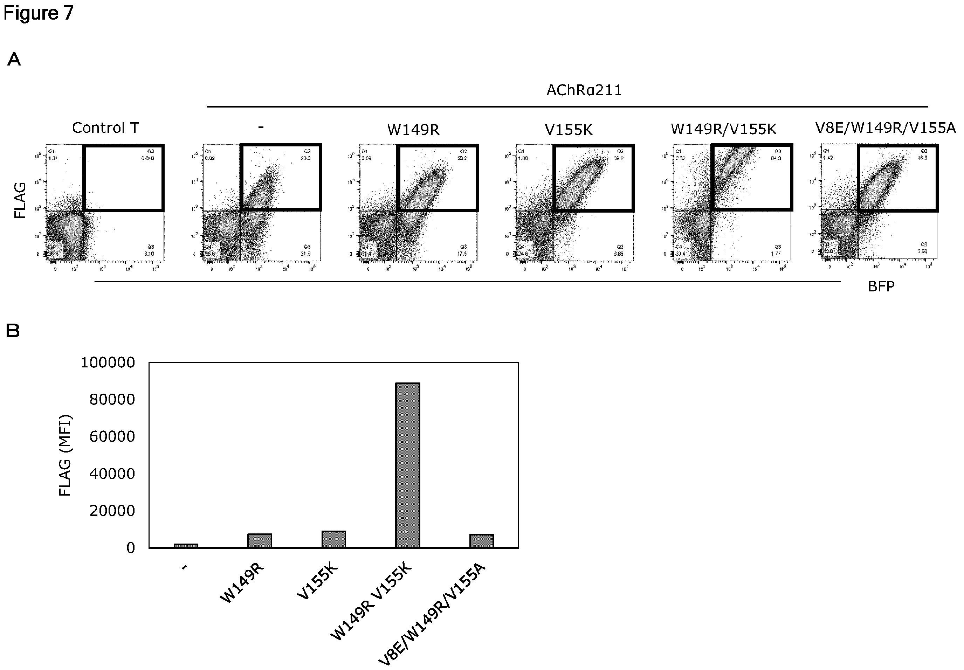

[Figure 7] The expression of AChRα-CAARs using an antigenic region having a W149R, V155K, W149R/V155K, or V8E/W149R/V155A mutation introduced in AChRα211 in human T cells was analyzed by flow cytometry. (A) The BFP was used as a reporter for gene transfer. Scatter plots are shown incorporating quartering lines based on BFP-negative cells as auxiliary lines, and BFP-positive and CAAR-positive cell fractions are delineated by thick lines. The X axis depicts the fluorescence intensity of BFP, and the Y axis depicts anti-FLAG antibody reaction. (B) BFP-positive cells are gated from each type of transfected T cells presented in (A), and anti-FLAG antibody reactivity is indicated by MFI.

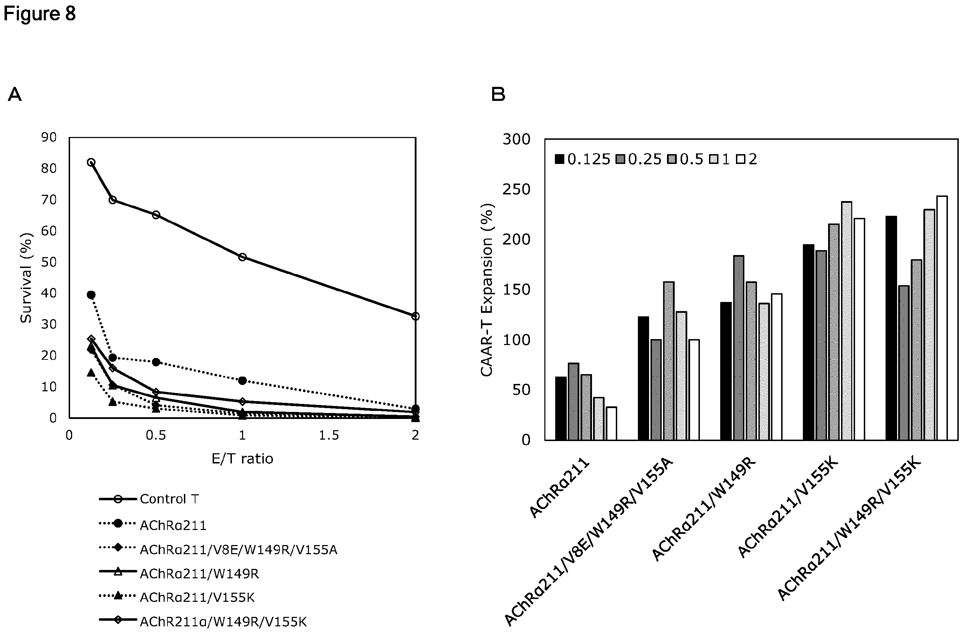

[Figure 8] Figure 8(A) is a line graph showing cytocidal activity of the CAAR-T cells prepared in Figure 7 against hybridomas: the X axis depicts an effector (CAAR-T cells)-target (hybridomas) mixed cell count (E/T ratio), and the Y axis depicts hybridoma cell survival as a percentage of the total number of hybridoma cells, wherein a reference of 100% is determined based onthe cell count of hybridomas without the addition of CAAR-T cells. Figure 8(B) is a bar graph showing the proliferation of each type of CAAR-T cells mentioned in the cytocidal tests of (A): the Y axis depicts proliferation, wherein a reference of 100% is determined based onthe cell count at the start of the reaction. In the graph, 2, 1, 0.5, 0.25, and 0.125 represent E/T ratios.

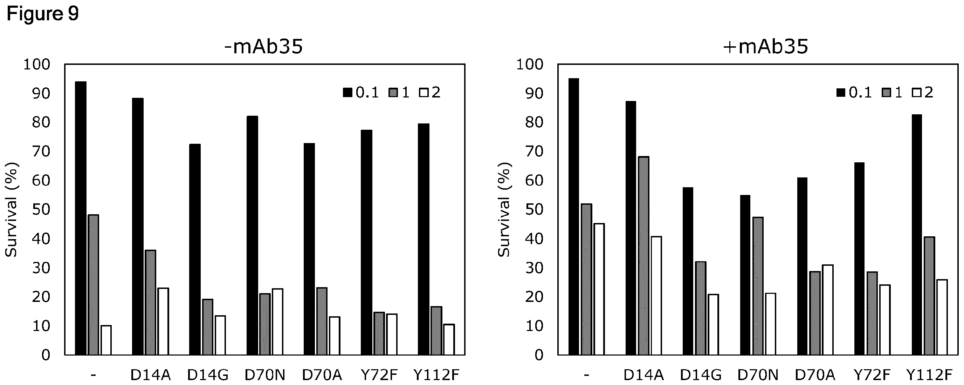

[Figure 9] AChRα-CAARs comprising an antigenic region having a D14A, D14G, D70N, D70A, Y72F, or Y112F mutation introduced in AChRα211/W149R/V155K were expressed in human T cells and evaluated for their cytocidal activity against membrane-type mAb35-scFv-expressing cells. The bar graph on the left shows data obtained in the absence of additional mAb35 antibody, and the bar graph on the right shows data obtained in the presence of additional mAb35 antibody. In the graphs, 2, 1, and 0.1 represent E/T (effector/target) ratios. The Y axis depicts survival wherein a reference of 100% was determined based on the cell count of target cells without the addition of CAAR-T cells.

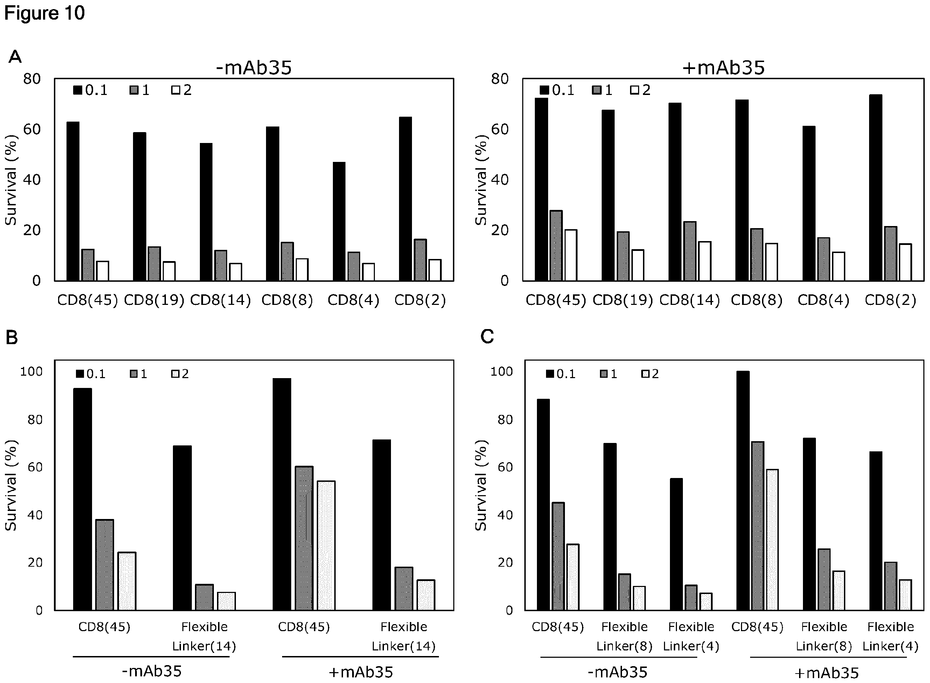

[Figure 10] AChRα-CAAR using AChRα211/W149R/V155K as an antigenic region and including a CD8α-derived linker (A) or a flexible linker (B & C) was expressed in human T cells and evaluated for its cytocidal activity against membrane-type mAb35-scFv-expressing cells. In the bar charts, 2, 1, and 0.1 represent E/T (effector/target) ratios. The Y axis depicts survival wherein a reference of 100% was determined based on the cell count of target cells without the addition of CAAR-T cells.

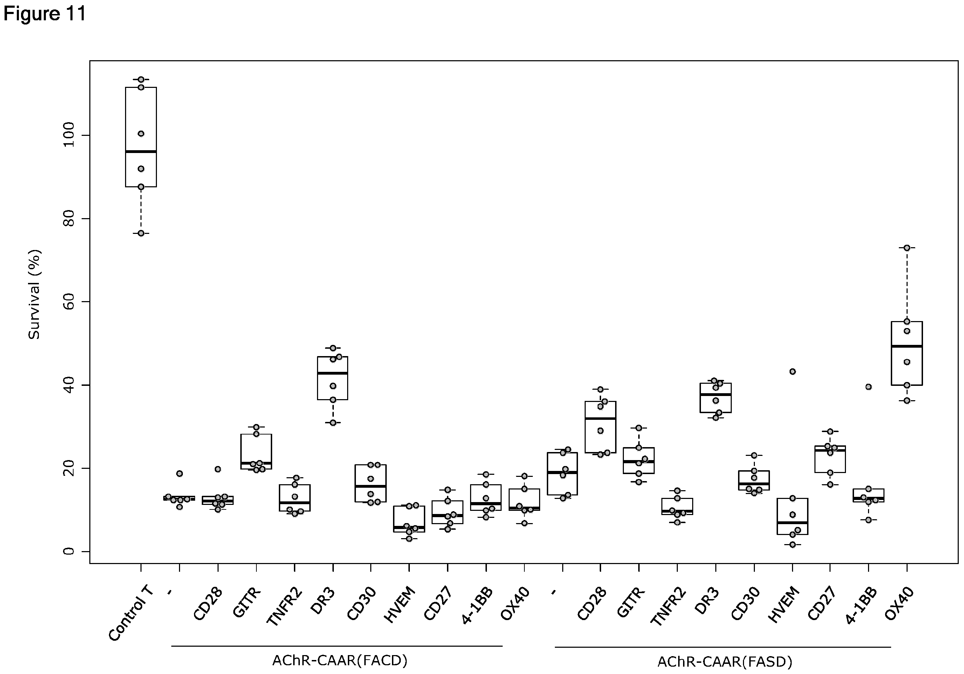

[Figure 11] T cells derived from multiple donors were transfected with a gene encoding AChRα-CAAR, which was constructed using AChRα211/W149R/V155K as an antigenic region,CD8α-derived four amino acids (FACD or FASD containing a CS mutation) as a linker region, and CD28, GITR, TNFR2, DR3, CD30, HVEM, CD27, 4-1BB, or OX40 as a co-stimulatory domain. Cytocidal activity against membrane-type mAb35-expressing RAJI cells is indicated as box-and-whisker plots. The Y axis depicts survival wherein a reference of 100% was determined based onthe cell count of target cells without the addition of CAAR-T cells.

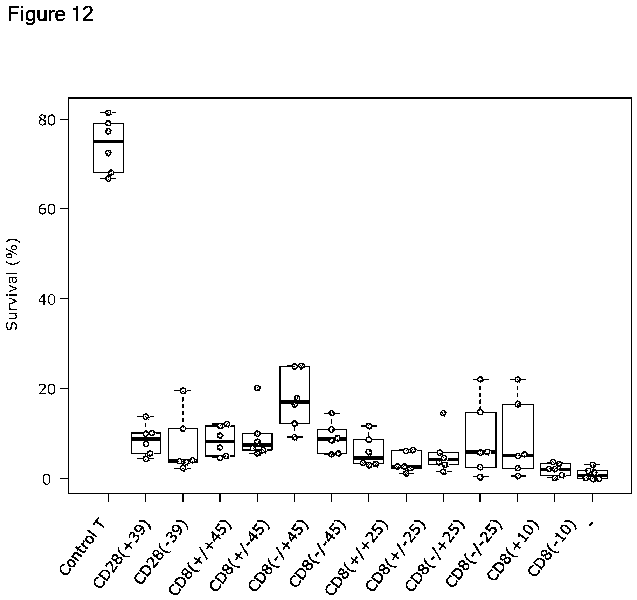

[Figure 12] Amino acids of a CD28- or CD8α-derived linker were variously deleted or mutated and provided as a linker region in AChRα-CAAR which included AChRα211/W149R/V155K as an antigenic region and HVEM as a co-stimulatory domain. T cells derived from multiple donors were transfected with the relevant gene encoding AChRα-CAAR to construct CAAR-T cells. Cytocidal activity against membrane-type mAb35-expressing RAJI cells are indicated in box-and-whisker plots. The X axis depicts the origin of the linker region in each AChRα-CAAR and the associated mutation, the numbers within parentheses represent the lengths of the linkers, and ± represents the site and number of substitutions of cysteine by serine (Table 6). The Y axis depicts survival wherein a reference of 100% was determined based onthe cell count of target cells without the addition of CAAR-T cells.

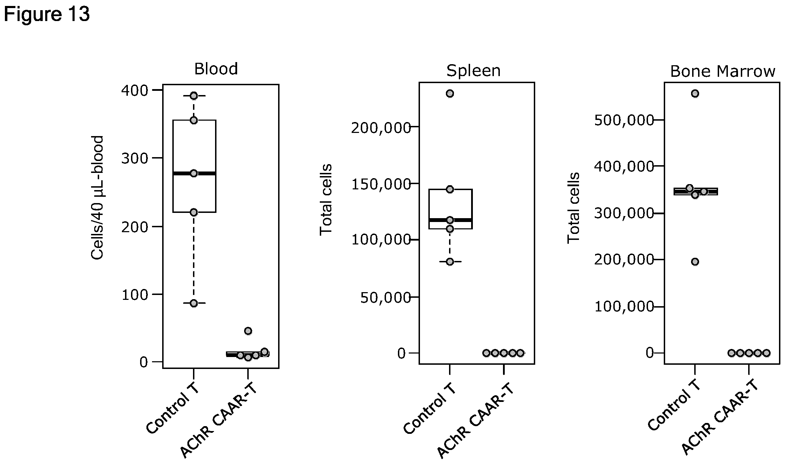

[Figure 13] Figure 13 shows box-and-whisker plots in which CAAR-T cells comprising AChRα211/W149R/V155K as an antigenic region were evaluated for their cytocidal activity against membrane-type mAb35-scFv-expressing RAJI cells using a xenograft model obtained using a NOG mouse. The plots show the cell count of membrane-type mAb35-scFv-expressing RAJI cells in 40 µL of blood (left), the whole spleen (middle), and the bone marrow of the femur and the tibia (right) 14 days after CAAR-T cell administration. Control T represents non-transfected T cells.

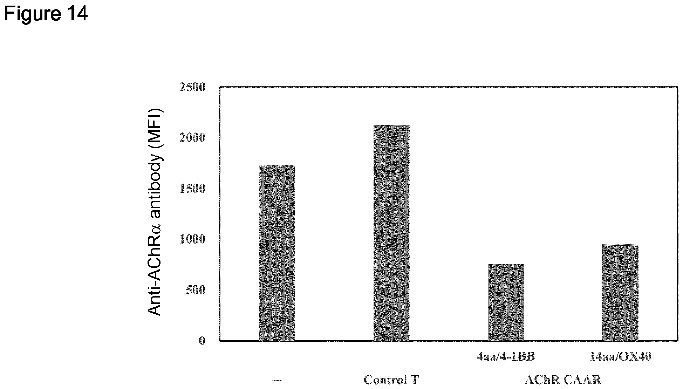

[Figure 14] Figure 14 shows the cytocidal activity of CAAR-T cells expressing AChRα-CAAR according to Example 12, against B cells producing anti-AChRα antibody obtained from the spleen of mice immunized with AChRα protein. The Y axis depicts MFI which represents the amount of an anti-AChRα antibody contained in the culture supernatant. Control T represents non-transfected T cells. "4aa/4-1BB" and "14aa/OX40" correspond to T cells expressing the AChRα-CAARs AChRα/W149R/V155K>4aa>4-1BB and AChRα/W149R/V155K>14aa>OX40, respectively.

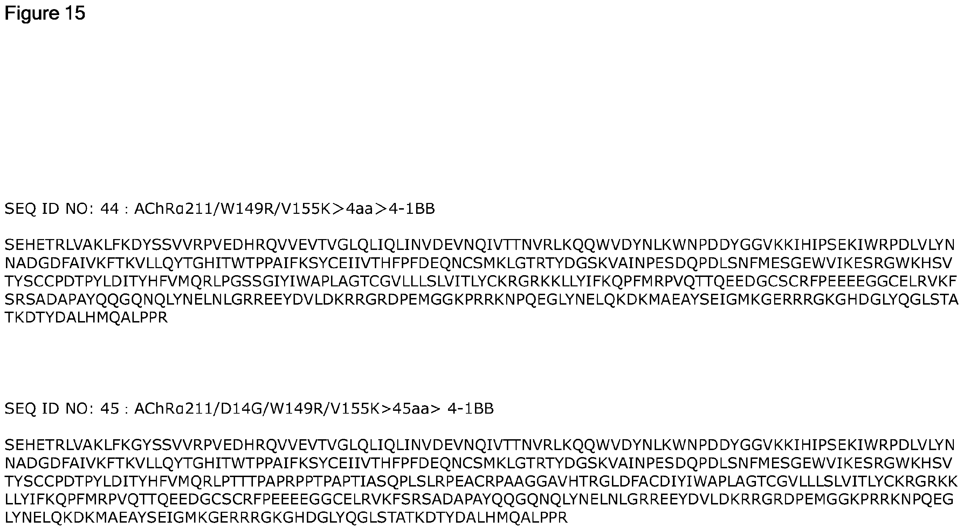

[Figure 15] Figure 15 shows the full-length amino acid sequence of two chimeric receptors of the present invention.

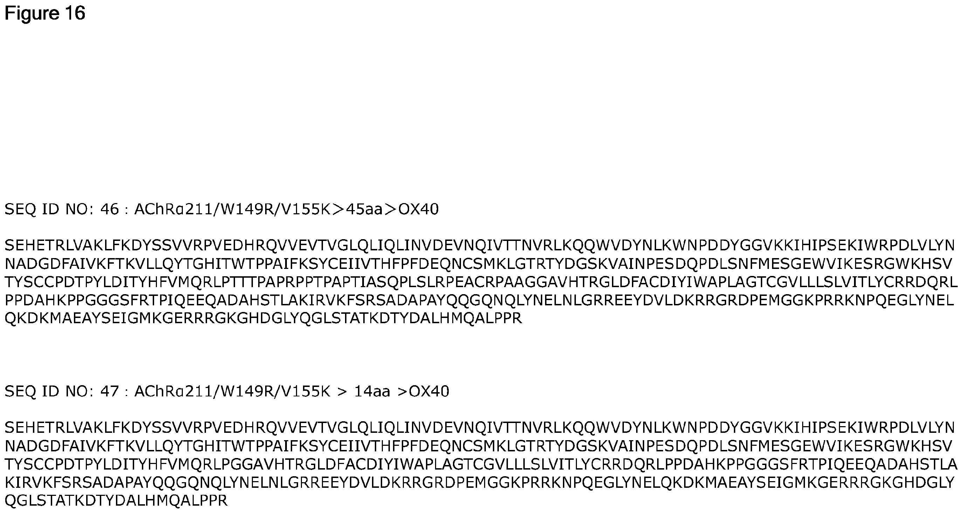

[Figure 16] Figure 16 shows the full-length amino acid sequence of a further two chimeric receptors of the present invention.

### Description of Embodiments

Hereinafter, the present invention will be described in detail.

### <Term>

In the present specification, the term "polypeptide" is a molecule in which two or more amino acids are linked through a peptide bond in a predetermined sequence. Its amino acid sequence comprises the amino acid sequence of an amino acid region, a domain, a peptide, or a protein having particular characteristics or functions. The term "polypeptide" as used herein has the same meaning as terms such as "protein", "peptide", and "oligopeptide. The term "isolated polypeptide" or "isolated protein" as used herein distinguishes the polypeptide or protein from a protein present *in vivo.* The isolated polypeptide or the isolated protein is not necessarily required to be physically separated or purified as a protein, and also includes a protein that is present in a cell culture supernatant or a tissue sample. The isolated polypeptide or the isolated protein means a polypeptide which is substantially free of cellular materials or culture media when produced by a recombinant DNA technique, or substantially free of chemical precursors or other chemicals when chemically synthesized.

In the present specification, the term "chimeric receptor" means a protein that functions as a chimeric autoantibody receptor for use in the field of autoimmune diseases, and it is also called CAAR. The CAAR is a protein having a structure with three regions, i.e., an extracellular region comprising an antigenic region capable of being bound by an antibody which binds to an autoantigen (autoantibody) and which is causative of an autoimmune disease, a transmembrane region, and an intracellular region comprising an intracellular signaling domain, where the three regions are arranged in that order from the N-terminus towards the C-terminus. A signal peptide may further be connected to the N-terminus of the antigenic region. In the extracellular region, a linker region may reside between the antigenic region and the transmembrane domain. In the intracellular region, an intracellular co-stimulatory domain may reside on the N-terminus of the intracellular signaling domain. The respective regions contained in the CAAR may be attached via an arbitrary spacer consisting of 0 to 10 amino acids.

In the present specification, the extracellular region is usually a region, of which the major part resides extracellularly when the chimeric receptor is expressed in a cell. Not all amino acids contained in the region necessarily reside extracellularly. Depending on the sequence of the extracellular region and any neighboring amino acid sequences, the arrangement of the extracellular domain may be such that a portion of the sequence corresponding to the extracellular region at the C-terminal end is buried in the cell membrane.

In the present specification, the transmembrane region is usually a domain, of which the major portion resides in a cell membrane when the chimeric receptor is expressed in a cell. Not all amino acids contained in the region necessarily reside in the cell membrane. Depending on the sequence of the transmembrane region and any neighboring amino acid sequences, the arrangement of the transmembrane region may by such that a portion of the sequence that corresponds to the transmembrane region at the N-terminal end may reside extracellularly and/or a portion of the sequence at the C-terminal end may reside intracellularly.

In the present specification, the intracellular region is usually a domain, of which the major resides intracellularly when the chimeric receptor is expressed in a cell. Not all amino acids contained in the domain necessarily reside intracellularly. Depending on the sequence of the intracellular region and any neighboring amino acid sequences, the arrangement of the intracellular region may be such that a portion of the sequence that corresponds to the intracellular region at the N-terminal end is buried in the cell membrane.

Various, well-known technologies applicable to chimeric antigen receptor (CAR)-T cells in the field of cancer treatment can readily be applied to the chimeric receptor of the present invention. For literature relating to the preparation of the CAR-T cells, see, for example, Uckun et al., 2011, Brit. J. Hematol., 153: 15-23; US 2012/0141505; and U.S. Patent Nos. 5,484,892, 5,573,924, 6,379,668, 7,744,877, 8,362,211, 9,023,999, 8,822,647, 9,328,156, and 9,034,324.

The antigenic region of the chimeric receptor has a structure that mimics at least a portion of an autoantigen (in the present invention, nAChRα1) causative of a targeted autoimmune disease, and has a function of specifically binding to the BCR expressed on pathogenic B cells and/or plasma cells that produce a targeted autoantibody, and transducing the binding signal to the intracellular signaling domain.

In the present specification, the term "nAChRα1" means a human nicotinic acetylcholine receptor α1 subunit or its extracellular region. This term may be used as a generic term including mutant nAChRα1 described below or it may be used to mean native nAChRα1, unless otherwise specified. The term "AchRα236" is used for distinguishing a native nAChRα1 extracellular region (SEQ ID NO: 1) consisting of 236 amino acids from a deletion-type nAChRα1 mentioned later.

In the present specification, the term "deletion-type nAChRα1" or "AchRα211" means nAChRα1 having the amino acid sequence as set forth in SEQ ID NO: 2 in which a region from positions 59 to 84 from the N-terminus is deleted from the amino acid sequence of AchRα236. SEQ ID NO:3 represents an amino acid sequence in which possible sites of substitutions in the deletion-type nAChRα1 are represented by Xaa. SEQ ID NO:4 represents AChRα236 and includes possible sites of substitutions represented by Xaa corresponding to those in SEQ ID NO:3 (mutant AChRα236).

In the present specification, the term "pathogenic B cells" mean B cells, plasma cells, and/or plasmablasts that produce an autoantibody that recognizes a self-antigen and that is causative of an autoimmune disease. Theautoantibodies that are produced bind to the cell surface of such pathogenic B cells, or a B cell receptor having the same antigen binding specificity as the autoantibody is expressed on the surface of the cells. In order to treat the autoimmune disease, the amount of pathogenic B cells in the body of a patient is decreased, and this, in turn, causes the level of thecorresponding autoantibody in the body to also decrease.

In the present specification, the term "amino acid" includes every native amino acid and modified amino acid. The term "conservative amino acid variation" is the substitution of one amino acid residue with another amino acid residue without impairing the desired functions or characteristics of the protein. Amino acids can be conservatively substituted for one another as long as their side chains have similar scientific and/or spatial characteristics. Such properties of amino acids have been well analyzed and are well known in the fields of biochemisty or molecular biology.

In the present specification, the term "hydrophobic amino acid" is an amino acid with a side chain having a low hydrophilicity or high hydrophobicity and is Val, Ala, Leu, Ile, Gly, Trp, Tyr, Phe, Met, or Pro as native amino acids. Among these, examples of hydrophobic amino acids having a bulky side chain include Trp, Tyr, and Phe. Examples of hydrophobic amino acids having a small side chain include Val, Ala, Leu, Ile, and Gly.

In the present specification, the term "hydrophilic amino acid" is an amino acid with a side chain having a high hydrophilicity and is Arg, Lys, Asp, Glu, Asn, Gln or Ser as native amino acids. An acidic amino acid is Asp or Glu. A basic amino acid is Arg or Lys. An amino acid having an amide group in its side chain is Asn or Gln.

In the present specification, the term "polynucleotide" means a nucleic acid in which nucleoside or nucleotide monomers consisting of native bases, sugars, and intersugar (backbone) bonds are linked according to a predetermined nucleotide sequence. This term also includes a modified or substituted sequence comprising a non-native monomer or a portion thereof. The polynucleotide of the present invention can be a deoxyribonucleic acid sequence (DNA), a ribonucleic acid sequence (RNA), or a DNA-RNA hybrid, and contains native bases including adenine, guanine, cytosine, thymidine and uracil. These sequences may also contain a modified base. Examples of such a modified base include aza and deaza adenine, guanine, cytosine, thymidine and uracil, and xanthine and hypoxanthine.

In the present specification, the term "isolated nucleic acid" means a nucleic acid substantially free of cellular materials or culture media when produced by a recombinant DNA technique, or substantially free of chemical precursors or other chemicals when chemically synthesized. The isolated nucleic acid is also substantially free of sequences, which in the corresponding native form, flank the nucleic acid (i.e., sequences positioned at the 5' and 3' ends of the nucleic acid) from which the isolated nucleic acid is derived. The term "nucleic acid" includes DNA and RNA, which can be either double-stranded or single-stranded, and corresponds to a sense or antisense strand. The term "nucleic acid" further includes a complementary nucleic acid sequence, for example, cDNA.

In the present specification, the term "cells" mean cells having a defined function, or a cell population comprising such cells. The cells may be a cell-containing sample isolated from their natural location or may be a cell population which is a mixture of different cells as long as the population includes cells that retain the defined function. The cells may be provided in a sample which may or may not have undergone separation or concentration, depending on the particular characteristics that are sought. Examples of the cells include, but are not limited to, cells collected from the living body (tissue cells, blood cells, lymphocytes, bone marrow cells, etc.), established cell lines, cells genetically engineered by transfection, somatic stem cells such as mesenchymal stem cells and hematopoietic stem cells, pluripotent stem cells such as ES cells and iPS cells, and cells obtained by the artificial induction or differentiation of somatic stem cells or pluripotent stem cells.

In the present specification, the term "genetically engineered cells" mean cells transfected with a polynucleotide encoding the chimeric receptor. The transfection method is not particularly limited, and various methods generally used in the fields of biogenetics or molecular biology can be applied. The cells to be engineered are not particularly limited. When the cells to be engineered are T cells, the genetically engineered cells are also called "CAAR-T cells" or "CAAR-T".

The genetically engineered cells of the present invention are typically administered to or transplanted into a patient suffering from an autoimmune disease. In autologous cell therapy, cells derived from the patient to be treated are used for administration or transplantation. In allogeneic cell therapy, the origin the cells to be administered or transplanted is not limited as long as the cells are insusceptible to immune rejection and can be used in allogeneic therapy. The cells may be single cells or may be a cell population which is a mixture of different cell types.

In the present specification, the term "T cells" mean cells having a biological, molecular biological, morphological, or genetic feature that allows them to be classified as T cells in this technical field, or a cell population comprising such cells. Examples of such features of T cells include the expression of a surface marker such as a T cell antigen receptor (TCR)/CD3 complex. A cell population that expresses a T cell marker as well as other markers corresponding to individual T cell populations (e.g., CD4, CD8, and TCR subtypes) and has a function which is comparable to other individual T cell populations may be used as the T cells.

The T cells may be provided as a cell population which has been separated or concentrated to obtain T cells which are positive for a T cell marker, or the T cells may be provided as a cell population in a biological sample (typically blood or a processed material thereof) containing T cells which has not been subjected to separation or concentration based on any T cell marker. Examples of such cell populations include blood cells, hemocytes, and peripheral blood mononuclear cells (PBMCs). A cell population obtained by inducing differentiation of somatic stem cells (hematopoietic stem cells) or pluripotent stem cells into T cells are also included as T cells.

In the present specification, the term "genetically engineered T cells" means the above-defined genetically engineered cells which may be T cells or a cell population comprising the T cells which are transfected. Genetically engineered T cells of the present invention include cells having a specific function such as individual types of T cells which have been obtained by separation or concentration using a T cell marker as well as other markers for individual T cell populations (e.g., CD4 and CD8).

In the present specification, the term "treatment" is an approach for obtaining beneficial or desired clinical results relating to health against a disease or a symptom. The beneficial or desired clinical results can include, but are not limited to, the alleviation or amelioration of one or more symptoms or pathological conditions, the diminishment of the extent of a disease, the stabilization (i.e., not worsening) of a disease state, the prevention of spread of a disease, the delay or slowing of progression of a disease, the amelioration or alleviation of a disease condition, and remission (partial or complete), regardless of whether the above characteristics are detectable or undetectable. The "treatment" may also mean the prolongation of a survival period compared with an expected survival period without the treatment. The term "alleviate" a disease or a disorder means that the extent and/or undesirable clinical manifestation of a disorder or a disease condition is lessened and/or the time course of progression is slowed or lengthened, as compared with the case of not treating the disorder.

In the present specification, the term "amino acid sequence or region derived from the amino acid sequence of a molecule or a region" means a sequence that consists of an amino acid sequence based on the sequence of the original molecule or region, optionally including a mutation of one or more amino acids, wherein the mutated sequence retains the function or activity of the original molecule or region above a certain level. The form of the amino acid mutation is not limited, and a mutation is accepted as long as the mutation is made through usual trial and error processes by those skilled in the art on the basis of the type, function, and sequence of the original molecule or region, and the mutation is rationally predicted to maintain the function or activity thereof. Examples of such mutations include, but are not limited to, a conservative amino acid substitution, a substitution by an amino acid having a similar function or physicochemical properties, a substitution, deletion, insertion, or addition of an amino acid in a region having a low contribution to a function or activity, and substitution, deletion, insertion, or addition of several amino acids at the N-terminal end or C-terminal end of the region. The number of amino acids to be mutated is usually 15 or fewer, preferably 0 or fewer, more preferably 7 or fewer, further preferably 5, 4, 3, 2, or 1.

### <Chimeric receptor and polypeptide>

The polypeptide of the present invention has an extracellular region comprising an antigenic region capable of being bound by an anti-nAChRα1 antibody, and the polypeptide is capable of functioning as a chimeric receptor. The antigenic region is a region comprising a deletion-type nAChRα1 extracellular region (SEQ ID NO: 2: AChRα211) consisting of 211 amino acids in which a region from positions 59 to 83 from the N-terminus of a native nAChRα1 extracellular region (SEQ ID NO: 1: AChRα236) consisting of 236 amino acids has been deleted, or a region comprising an amino acid sequence derived from the amino acid sequence as set forth in SEQ ID NO: 2 by the substitution, deletion, insertion, and/or addition (also collectively referred to as "mutation") of one or several amino acids.

Examples of the amino acids to be substituted in SEQ ID NO: 2 can include amino acids represented by Xaa in SEQ ID NO: 3 or SEQ ID NO: 42. However, the site to be mutated is not limited thereto as long as the function of the chimeric receptor is retained.

In the present specification, amino acid substitution mutations are represented by an indication of the original amino acid before the amino acid position of interest in SEQ ID NO: 2, and an indication of the substituting amino acid after the amino acid position of interest These amino acids are each indicated by a single-letter code. A plurality of amino acid mutations, if included, are represented by the respective mutations delimited by "/". For example, an antigenic region in which Val at position 8 of SEQ ID NO: 2 is substituted with Glu, Trp at position 149 is substituted with Arg, and Val at position 155 is substituted with Ala can be represented by "AChRα211/V8E/W149R/V155A".

When amino acid Xaa8 of SEQ ID NO: 3 is Val or Glu, the resulting polypeptide has been confirmed to function as CAAR. Therefore, its substitution with diverse amino acids is accepted. The substitution is preferably with a hydrophobic amino acid or an acidic amino acid, more preferably by a hydrophobic amino acid having a small side chain or an acidic amino acid, further preferably with Val or Glu, most preferably with Val.

When amino acid Xaa14 of SEQ ID NO: 3 or SEQ ID NO: 42 is Asp, Ala, or Gly, the resulting polypeptide has been confirmed to function as CAAR. Therefore, its substitution with diverse amino acids is accepted. The substitution is preferably with an acidic amino acid or a hydrophobic amino acid, more preferably with an acidic amino acid or a hydrophobic amino acid having a small side chain, further preferably withAsp, Ala, or Gly.

When amino acid Xaa70 of SEQ ID NO: 3 is Asp, Asn, or Ala, the resulting polypeptide has functioned as CAAR. Therefore, its substitution with diverse amino acids is accepted. The substitution is preferably by an acidic amino acid, an amino acid having a side chain comprising an amide group, or a hydrophobic amino acid, more preferably Asp, Asn, or Ala.

When amino acid Xaa72 of SEQ ID NO: 3 is Tyr or Phe, the resulting polypeptide has been confirmed to function as CAAR. Therefore, its substitution with diverse amino acids is accepted. The substitution is preferably with a hydrophobic amino acid, more preferably with a hydrophobic amino acid having a bulky side chain, and further preferably withTyr or Phe.

When amino acid Xaa112 of SEQ ID NO: 3 is Tyr or Phe, the resulting polypeptide has been confirmed to function as CAAR. Therefore, its substitution with diverse amino acids is accepted. The substitution is preferably with a hydrophobic amino acid, more preferably with a hydrophobic amino acid having a bulky side chain, further preferably Tyr or Phe.

When amino acid Xaa149 of SEQ ID NO: 3 or SEQ ID NO: 42 is Trp, Arg, Ala, Lys, Asp, Ser, Gly, Asn, Ile, Phe, Met, or Pro, the resulting polypeptide has been confirmed to function as CAAR. Therefore, its substitution with diverse amino acids is accepted. The substitution is preferably with a hydrophilic amino acid or a hydrophobic amino acid, more preferably withTrp, Arg, Ala, Lys, Asp, Ser, Gly, Asn, Ile, Phe, Met, or Pro, and further preferably withTrp, Arg, Lys, or Pro.

When amino acid Xaa155 of SEQ ID NO: 3 or SEQ ID NO: 42 is Val, Arg, Lys, Asp, Ser, Gly, Asn, Ile, Phe, Met, or Pro, the resulting polypeptide has been confirmed to function as CAAR. Therefore, its substitution with diverse amino acids is accepted. The substitution is preferably with a hydrophobic amino acid or a hydrophilic amino acid, more preferably withTrp, Arg, Ala, Lys, Asp, Ser, Gly, Asn, Ile, Phe, Met, or Pro, and further preferably withVal, Lys, Ala, Met, Asp, Pro, or Gly.

A region corresponding to amino acid positions 146 to 159 of SEQ ID NO: 2 is a region rich in hydrophobic amino acids. Even when the respective amino acids of the region are appropriately substituted with Lys, a polypeptide having the resulting sequence has been confirmed to function as CAAR. Therefore, in the region corresponding to Xaa146 to Xaa159 of SEQ ID NO: 3, the substitution of amino acids other than Xaa149 and Xaa155 by diverse amino acids is possible. The substitution is preferably with a basic amino acid or a hydrophobic amino acid, more preferably with an amino acid of the corresponding site in SEQ ID NO: 2 or Lys. Specific examples of the amino acid sequence of the region from Xaa146 to Xaa159 of SEQ ID NO: 3 include the sequences as set forth in SEQ ID NO: 16 to SEQ ID NO: 23.

Even when each of Xaa192 and Xaa193 (Cys in the original sequence) of SEQ ID NO: 3 is substituted with Gly, the resulting polypeptide has been confirmed to function as CAAR. Therefore, their substitution with diverse amino acids is possible. Each of the amino acids may independently be retained as Cys or substituted with Gly, preferably at least one of the amino acids is retained as Cys and the other amino acid is substituted with Gly, and further preferably, both are substituted with Gly.

One form of the antigenic region in the chimeric receptor of the present invention is a region comprising the amino acid sequence of SEQ ID NO: 3, wherein Xaa14 is Asp, Ala, or Gly, Xaa149 is Trp, Arg, Lys, or Pro, and Xaa155 is Lys, Ala, Met, Asp, Pro, or Gly.

In this case, the other amino acids Xaa are selected from the amino acids described above and are preferably as similar as possible to those of the sequence of native nAChRα1 in order to bind to pathogenic B cells. Hence, the number of substituted amino acids Xaa other than Xaa14, Xaa149, and Xaa155 in SEQ ID NO: 3 are preferably 10 or less, more preferably 5 or less, further preferably 3, 2, or 1, most preferably 0 (i.e., the amino acids Xaa other than Xaa14, Xaa149, and Xaa155 are the corresponding ones in SEQ ID NO: 2 such that the amino acid sequence of the antigenic region corresponds to SEQ ID NO: 42). Xaa other than Xaa149 and Xaa155 in SEQ ID NO: 3, when substituted, is preferably selected from the group consisting of Xaa8, Xaa70, Xaa72, Xaa192, and Xaa193, more preferably selected from the group consisting of Xaa8, Xaa192, and Xaa193.

Specific examples of the antigenic region contained in the chimeric receptor of the present invention can include AChRα211, AChRα211/V8E, AChRα211/W149R, AChRα211/W149K, AChRα211/W149P, AChRα211/V155A, AChRα211/V155K, AChRα211/V155M, AChRα211/V155D, AChRα211/V155P, AChRα211/C192G, AChRα211/C193G, AChRα211/C192G/C193G, AChRα211/V8E/W149R, AChRα211/V8E/W149K, AChRα211/V8E/W149P, AChRα211/V8E/V155A, AChRα211/V8E/V155K, AChRα211/V8E/V155M, AChRα211/V8E/V155D, AChRα211/V8E/V155P, AChRα211/V8E/C192G, AChRα211/V8E/C193G, AChRα211/V8E/C192G/C193G, AChRα211/W149R/V155A, AChRα211/W149R/V155K, AChRα211/W149R/V155M, AChRα211/W149R/V155D, AChRα211/W149R/V155P, AChRα211/W149R/C192G, AChRα211/W149R/C193G, AChRα211/W149R/C192G/C193G, AChRα211/W149K/V155A, AChRα211/W149K/V155K, AChRα211/W149K/V155M, AChRα211/W149K/V155D, AChRα211/W149K/V155P, AChRα211/W149K/C192G, AChRα211/W149K/C193G, AChRα211/W149K/C192G/C193G, AChRα211/W149P/V155A, AChRα211/W149P/V155K, AChRα211/W149P/V155M, AChRα211/W149P/V155D, AChRα211/W149P/V155P, AChRα211/W149P/C192G, AChRα211/W149P/C193G, AChRα211/W149P/C192G/C193G, AChRα211/V8E/W149R/V155A, AChRα211/V8E/W149R/V155K, AChRα211/V8E/W149R/V155M, AChRα211/V8E/W149R/V155D, AChRα211/V8E/W149R/V155P, AChRα211/V8E/W149R/C192G, AChRα211/V8E/W149R/C193G, AChRα211/V8E/W149R/C192G/C193G, AChRα211/V8E/W149K/V155A, AChRα211/V8E/W149K/V155K, AChRα211/V8E/W149K/V155M, AChRα211/V8E/W149K/V155D, AChRα211/V8E/W149K/V155P, AChRα211/V8E/W149K/C192G, AChRα211/V8E/W149K/C193G, AChRα211/V8E/W149K/C192G/C193G, AChRα211/V8E/W149P/V155A, AChRα211/V8E/W149P/V155K, AChRα211/V8E/W149P/V155M, AChRα211/V8E/W149P/V155D, AChRα211/V8E/W149P/V155P, AChRα211/V8E/W149P/C192G, AChRα211/V8E/W149P/C193G, AChRα211/V8E/W149P/C192G/C193G, AChRα211/W149R/V155A/C192G, AChRα211/W149R/V155K/C192G, AChRα211/W149R/V155M/C192G, AChRα211/W149R/V155D/C192G, AChRα211/W149R/V155P/C192G, AChRα211/W149K/V155A/C192G, AChRα211/W149K/V155K/C192G, AChRα211/W149K/V155M, /C192G, AChRα211/W149K/V155D/C192G, AChRα211/W149K/V155P/C192G, AChRα211/W149P/V155A/C192G, AChRα211/W149P/V155K/C192G, AChRα211/W149P/V155M, /C192G, AChRα211/W149P/V155D/C192G, AChRα211/W149P/V155P/C192G AChRα211/W149R/V155A/C193G, AChRα211/W149R/V155K/C193G, AChRα211/W149R/V155M/C193G, AChRα211/W149R/V155D/C193G, AChRα211/W149R/V155P/C193G, AChRα211/W149K/V155A/C193G, AChRα211/W149K/V155K/C193G, AChRα211/W149K/V155M/C193G, AChRα211/W149K/V155D/C193G, AChRα211/W149K/V155P/C193G, AChRα211/W149P/V155A/C193G, AChRα211/W149P/V155K/C193G, AChRα211/W149P/V155M/C193G, AChRα211/W149P/V155D/C193G, AChRα211/W149P/V155P/C193G, AChRα211/W149R/V155A/C192G/C193G, AChRα211/W149R/V155K/C192G/C193G, AChRα211/W149R/V155M/C192G/C193G, AChRα211/W149R/V155D/C192G/C193G, AChRα211/W149R/V155P/C192G/C193G, AChRα211/W149K/V155A/C192G/C193G, AChRα211/W149K/V155K/C192G/C193G, AChRα211/W149K/V155M/C192G/C193G, AChRα211/W149K/V155D/C192G/C193G, AChRα211/W149K/V155P/C192G/C193G, AChRα211/W149P/V155A/C192G/C193G, AChRα211/W149P/V155K/C192G/C193G, AChRα211/W149P/V155M/C192G/C193G, AChRα211/W149P/V155D/C192G/C193G, AChRα211/W149P/V155P/C192G/C193G, AChRα211/D14G/W149R/V155K, AChRα211/D14G/W149R/V155A, AChRα211/D14G/W149R/V155M, AChRα211/D14G/W149R/V155D, AChRα211/D14G/W149R/V155P, AChRα211/D14G/W149R/V155G, AChRα211/D14G/W149K/V155K, AChRα211/D14G/W149K/V155A, AChRα211/D14G/W149K/V155M, AChRα211/D14G/W149K/V155D, AChRα211/D14G/W149K/V155P, AChRα211/D14G/W149K/V155G, AChRα211/D14G/W149P/V155K, AChRα211/D14G/W149P/V155A, AChRα211/D14G/W149P/V155M, AChRα211/D14G/W149P/V155D, AChRα211/D14G/W149P/V155P, AChRα211/D14G/W149P/V155G, AChRα211/D14A/W149R/V155K, AChRα211/D14A/W149R/V155A, AChRα211/D14A/W149R/V155M, AChRα211/D14A/W149R/V155D, AChRα211/D14A/W149R/V155P, AChRα211/D14A/W149R/V155G, AChRα211/D14A/W149K/V155K, AChRα211/D14A/W149K/V155A, AChRα211/D14A/W149K/V155M, AChRα211/D14A/W149K/V155D, AChRα211/D14A/W149K/V155P, AChRα211/D14A/W149K/V155G, AChRα211/D14A/W149P/V155K, AChRα211/D14A/W149P/V155A, AChRα211/D14A/W149P/V155M, AChRα211/D14A/W149P/V155D, AChRα211/D14A/W149P/V155P, and AChRα211/D14A/W149P/V155G.

The antigenic region to be included in the chimeric receptor of the present invention may have a substitution, deletion, insertion, and/or addition of one or several amino acids (hereinafter, referred to as "additional mutation"), in addition to the substitution of an amino acid represented by Xaa in SEQ ID NO: 3, as long as the chimeric receptor is expressed in a cell membrane and has a function of being recognized by an anti-nAChRα1 antibody. Such an additional mutation may be made at any position of SEQ ID NO: 2 and is preferably made in a region within 10 amino acids from the N-terminus and/or the C-terminus, more preferably a site within 5, 4, 3, 2, or 1 amino acids therefrom. The number of amino acids to be mutated is 10 or fewer, preferably 5, 4, 3, 2, or 1. The form of the additional mutation in the antigenic region is preferably the deletion or addition of 10 or fewer amino acids a the N-terminal and/or C-terminal ends of SEQ ID NO: 2, more preferably the deletion or addition of 5, 4, 3, 2, or 1 amino acids at the C-terminal end, further preferably the addition of 5, 4, 3, 2, or 1 amino acids at the C-terminal end.

The ability of CAAR comprising the antigenic region of the present invention to bind to an anti-human nAChRα1 antibody can be confirmed by testing, in accordance with methods known in the art, and the binding of an anti-nAChRα1 antibody to cells transfected with a polynucleotide encoding the CAAR may be confirmed by methods mentioned below. Examples of such methods can include FACS and immunostaining using an anti-nAChRα1 antibody. The antibody used can be a commercially available anti-nAChRα1 antibody, or a recombinant protein comprising an antigen binding site of such an antibody can be used. Such a recombinant protein can be appropriately prepared with reference to the amino acid sequence of mAb35 antibody (SEQ ID NO: 33: membrane-type mAb35, SEQ ID NO: 35: mAb35 hIgG1, SEQ ID NO: 36: mAb35 hIgGκ) known as an anti-nAChRα1 antibody. For example, an antibody-containing sample such as a blood sample of a patient with MG or a sample of an antibody component separated or concentrated from the sample can be used. An anti-nAChRα1 antibody obtained from the blood of a patient with MG has been reported to compete with mAb35 for binding to nAChRα1 (Non Patent Literature 5: Luo et al., J Neurosci. 2009 Nov 4; 29 (44): 13898-908.). mAb35 is considered to recognize a similar epitope to that recognized by a patient's autoantibody. Hence, CAAR having an antigenic region capable of being bound by mAb35 is considered to bind to pathogenic B cells.

The chimeric receptor of the present invention may comprise a signal peptide for enabling expression in a cell membrane. The N-termini of many secretory proteins and membrane proteins comprise a signal peptide, which has, but which is not limited to, a length of the order of approximately 15 to 30 amino acids. Such a signal peptide is not limited by the form of linkage or the type of sequence as long as the signal peptide does not inhibit the function of the chimeric receptor and it is in a form that permits expression in a cell membrane. A preferred point of linkage of the signal peptide is at the N-terminus of the chimeric receptor. Specific examples of the signal peptide can include a CD8α-derived signal peptide (SEQ ID NO: 9), an nAChRα1-derived signal peptide (SEQ ID NO: 10), and a human immunoglobulin-derived signal peptide (SEQ ID NO: 11). An amino acid sequence comprising a portion of the above sequences or the whole sequences may be adopted as a signal peptide.

The extracellular region in the chimeric receptor of the present invention may have a linker region that consists of approximately 300 or less amino acids and that functions to link the antigenic region to the transmembrane region. Various sequences can be used for the linker region providing the specific binding of the antigenic region to a target pathogenic B cell is not markedly inhibited. The sequence may be a sequence derived from a linker or hinge region derived from a native protein (e.g., a hinge region of CD8, CD28, or the like, a hinge region derived from each IgG, and a hinge region consisting of 4 to 8 amino acids (short hinge) are described in literature known in the art such as Non Patent Literature 1), or the linker may be an artificially designed sequence. The amino acid length of the linker region is, for example, 300 amino acids or less, 100 amino acids or less, or approximately 50 amino acids or less. As shown in Examples 8 and 10 mentioned below, even when the linker region of a chimeric receptor of the present invention was mutated in different ways, so as to provide linker sequences such as sequences of varying lengths comprising two or more amino acids obtained by deletion of amino acid residues at the N-terminus of a sequence derived from a hinge region of CD8α (SEQ ID NO: 39) or CD28 (SEQ ID NO: 40), or the artificially designed linker sequence of SEQ ID NO: 41, or a sequence derived from such a linker sequence by the substitution of Cys that contributes to a disulfide bond with Ser, or to provide a chimeric receptor free of the linker region, the chimeric receptor on the cell membrane maintained the ability to bind to an anti-nAChRα1 antibody. Therefore, it has been confirmed that the linker region is not limited by a particular sequence or length and various amino acid sequences are possible.

The linker region preferably promotes the binding of the antigenic region to an autoantibody or a BCR on pathogenic B cells, and has a sequence that enhances signal transduction to cells. Examples of amino acids which are expected to promote the binding include Cys, charged amino acids, and Ser and Thr within a latent glycosylation site. These amino acids can be used as amino acids that constitute the linker region.

In one aspect of the present invention, the linker region contained in the chimeric receptor is an amino acid sequence derived from amino acid positions 118 to 178 of CD8α (NCBI RefSeq: NP_001759.3), i.e., a CD8α hinge region, amino acid positions 135 to 195 of CD8β (GenBank: AAA35664.1), amino acid positions 315 to 396 of CD4 (NCBI RefSeq: NP_000607.1), amino acid positions 114 to 152 of CD28 (NCBI RefSeq: NP_006130.1), or at least a portion thereof. The sequence of the linker region is preferably an amino acid sequence comprising a sequence of four or more consecutive amino acids contained in the CD8α hinge region as set forth in SEQ ID NO: 5 or SEQ ID NO: 39 (more preferably an amino acid sequence corresponding to amino acid positions 42 to 45, amino acid positions 38 to 45, amino acid positions 36 to 45, amino acid positions 32 to 45, amino acid positions 27 to 45, amino acid positions 21 to 45, or amino acid positions 1 to 45), or an amino acid sequence comprising four or more consecutive amino acids contained in the CD28 hinge region as set forth in SEQ ID NO: 40 (more preferably amino acid positions 36 to 39, amino acid positions 30 to 39, amino acid positions 20 to 39, or amino acid positions 1 to 39), or a sequence of four or more consecutive amino acids contained in a region in the flexible linker as set forth in SEQ ID NO: 41 (preferably an amino acid sequence corresponding to amino acid positions 11 to 14, amino acid positions 8 to 14, amino acid positions 4 to 14, or amino acid positions 1 to 14).

The transmembrane region contained in the chimeric receptor of the present invention is not particularly limited as long as the transmembrane region resides across a cell membrane. The transmembrane region may be derived from a native membrane-bound proteins or may be artificially designed. Examples of transmembrane region which may be included in the chimeric receptor of the present invention include, but are not limited to, amino acid sequences derived from transmembrane domains of nAChRα1, T cell receptor α or β chain, CD3ζ chain, CD28, CD3ε, CD45, CD4, CD5, CD8, CD9, CD16, CD22, CD33, CD27, CD64, CD80, CD86, CD134, CD137, ICOS, CD154, GITR, or the like. A CD8 α transmembrane domain (SEQ ID NO: 6), a CD28 transmembrane domain (SEQ ID NO: 37), or an nAChRα1 transmembrane domain (SEQ ID NO: 38) is preferred. The artificially designed transmembrane region typically has an amino acid sequence comprising hydrophobic amino acids. For example, a triplet of Phe, Trp, and Val can be found at each end of a synthesized transmembrane region.

In another aspect, when the chimeric receptor of the present invention includes a linker region comprising at least a region of the C-terminus of a hinge region of a native membrane-type protein, the linker region and the transmembrane region to be included in this chimeric receptor can be derived from the same protein (e.g., a combination of a hinge region and a transmembrane domain of CD8α, or a combination of a hinge region and a transmembrane domain of CD28). As a result, the structure near a cell membrane of the extracellular region can be a structure similar to that of a native protein.

The chimeric receptor of the present invention can be designed to form a multimer, particularly, a dimer. For example, Cys may be inserted to the linker region and/or the transmembrane region in order to multimerize (dimerize) chimeric receptors through a disulfide bond. In Example 10 mentioned below, CD8α and CD28-derived hinge regions were used as linker regions, and it was confirmed that even when Cys contained in the hinge regions was substituted with Ser (Xaa27 and Xaa44 of SEQ ID NO: 39 or Xaa28 of SEQ ID NO: 40), the function of the CAAR was maintained.

The intracellular region in the chimeric receptor of the present invention has an intracellular signaling domain for transducing a signal arising from the binding of an autoantibody to the antigenic region into cells, and activating the cytotoxic activity of T cells expressing the chimeric receptor. The domain is not particularly limited, and any sequence derived from a domain typically used in the field of CAR-T technology can be applied thereto. Representative examples of the domain of origin can include a CD3ζ intracellular signaling domain (SEQ ID NO: 8) and a CD3δ intracellular signaling domain.

The intracellular region in the chimeric receptor of the present invention may have an intracellular co-stimulatory domain in addition to the intracellular signaling domain, for further enhancing the signal. The region is not particularly limited, and any sequence derived from an intracellular domain or a co-stimulatory domain of a molecule typically used in the field of CAR-T technology or a molecule that is expressed on a T cell surface and known to enhance the activation of T cells can be applied thereto. Examples of the intracellular co-stimulatory domain include sequences derived from intracellular domains of CD2, CD4, CD5, CD8α, CD8β, CD28, CD134, CD137 (4-1BB), ICOS, GITR, TNFR2, DR3, CD30, HVEM, CD27, OX40, and CD154, terminally truncated fragments thereof comprising a signaling motif, and co-stimulatory domains thereof. Specific examples of the above-mentioned co-stimulatory domains include sequences corresponding to amino acid positions 236 to 351 of CD2 (NCBI RefSeq: NP_001758.2), amino acid positions 421 to 458 of CD4 (NCBI RefSeq: NP_000607.1), amino acid positions 402 to 495 of CD5 (NCBI RefSeq: NP_055022.2), amino acid positions 207 to 235 of CD8α (NCBI RefSeq: NP_001759.3), amino acid positions 196 to 210 of CD8β (GenBank: AAA35664.1), amino acid positions 180 to 220 of CD28 (NCBI RefSeq: NP_006130.1), amino acid positions 214 to 255 of CD137 (4-1BB, NCBI RefSeq: NP_001552.2), amino acid positions 241 to 277 of CD134 (OX40, NCBI RefSeq: NP_003318.1), and amino acid positions 166 to 199 of ICOS (NCBI RefSeq: NP_036224.1). Other specific examples thereof can include a 4-1BB co-stimulatory domain (SEQ ID NO: 7), a CD28 co-stimulatory domain (SEQ ID NO: 25), a GITR co-stimulatory domain (SEQ ID NO: 26), a TNFR2 co-stimulatory domain (SEQ ID NO: 27), a DR3 co-stimulatory domain (SEQ ID NO: 28), a CD30 co-stimulatory domain (SEQ ID NO: 29), an HVEM co-stimulatory domain (SEQ ID NO: 30), a CD27 co-stimulatory domain (SEQ ID NO: 31), and an OX40 co-stimulatory domain (SEQ ID NO: 32).

When a co-stimulatory domain is included in the chimeric receptor of the present invention, one or more (for example, two or three) sequences derived from any one of the molecules described above may be included. When more than one sequence is included, the sequences may be combined arbitrarily. Examples of such combinations include a combination of co-stimulatory domains derived from CD28 and 4-1BB known in the CAR-T field.

The chimeric receptor of the present invention has been confirmed to function regardless of the type of the co-stimulatory domain because CAARs that included co-stimulatory domains derived from various membrane proteins were confirmed to function appropriately in Example 9 mentioned below. The molecule from which the co-stimulatory domain of the present invention is derived is preferably 4-1BB, CD28, GITR, TNFR2, DR3, CD30, HVEM, CD27, or OX40.

The chimeric receptor of the present invention may include a further region or a sequence known in the field of CAR-T technology or other biotechnological fields in addition to the antigenic region. Each sequence described in the present specification corresponding to a further region is a representative sequence of the region, and some representative sequences have a mutation of one or several amino acids. The term "sequence derived from" such a sequence known in the art means a sequence identical to the sequence known in the art, or a sequence that is derived from the sequence known in the art by the introduction of a mutation of one or several amino acids, whilst retaining a function of the relevant region. When such a sequence derived from a region known in the art contains an amino acid mutation, the location of the mutation is not particularly limited providing a function of the region is retained. The mutation preferably involves an amino acid that falls outside the functional center of the region.

In the chimeric receptor of the present invention, a spacer sequence consisting of one or more linking amino acids may be inserted between the respective regions. For example, a spacer sequence of 2 to 10 amino acids in length can be used. Particularly, a spacer sequence having a glycine-serine consecutive sequence can be used.

The chimeric receptor of the present invention is, for example, a polypeptide comprising the following regions from the N-terminus towards the C-terminus: an antigenic region comprising a sequence represented by the AChRα211 of SEQ ID NO: 2 or the mutant AChRα211 of SEQ ID NO: 3 or SEQ ID NO: 42(wherein unsubstituted Xaa is an amino acid at the corresponding position in SEQ ID NO: 2); a transmembrane region consisting of a sequence derived from a transmembrane domain of any one domain selected from nAChRα1, T cell receptor α chain, T cell receptor β chain, CD3ζ chain, CD28, CD3ε, CD45, CD4, CD5, CD8, CD9, CD16, CD22, CD33, CD37, CD64, CD80, CD86, CD134, CD137, ICOS, CD154, and GITR; and an intracellular region comprising a sequence derived from an intracellular signaling domain of CD3ζ or CD3δ; preferably a polypeptide comprising from the N-terminus towards the C-terminus: an antigenic region selected from AChRα211/W149R/V155K, AChRα211/W149R/V155A, AChRα211/W149R/V155M, AChRα211/W149R/V155D, AChRα211/W149R/V155P, AChRα211/W149R/V155G, AChRα211/W149K/V155K, AChRα211/W149K/V155A, AChRα211/W149K/V155M, AChRα211/W149K/V155D, AChRα211/W149K/V155P, AChRα211/W149K/V155G, AChRα211/W149P/V155K, AChRα211/W149P/V155A, AChRα211/W149P/V155M, AChRα211/W149P/V155D, AChRα211/W149P/V155P, AChRα211/W149P/V155G, AChRα211/D14G/W149R/V155K, AChRα211/D14G/W149R/V155A, AChRα211/D14G/W149R/V155M, AChRα211/D14G/W149R/V155D, AChRα211/D14G/W149R/V155P, AChRα211/D14G/W149R/V155G, AChRα211/D14G/W149K/V155K, AChRα211/D14G/W149K/V155A, AChRα211/D14G/W149K/V155M, AChRα211/D14G/W149K/V155D, AChRα211/D14G/W149K/V155P, AChRα211/D14G/W149K/V155G, AChRα211/D14G/W149P/V155K, AChRα211/D14G/W149P/V155A, AChRα211/D14G/W149P/V155M, AChRα211/D14G/W149P/V155D, AChRα211/D14G/W149P/V155P, AChRα211/D14G/W149P/V155G, AChRα211/D14A/W149R/V155K, AChRα211/D14A/W149R/V155A, AChRα211/D14A/W149R/V155M, AChRα211/D14A/W149R/V155D, AChRα211/D14A/W149R/V155P, AChRα211/D14A/W149R/V155G, AChRα211/D14A/W149K/V155K, AChRα211/D14A/W149K/V155A, AChRα211/D14A/W149K/V155M, AChRα211/D14A/W149K/V155D, AChRα211/D14A/W149K/V155P, AChRα211/D14A/W149K/V155G, AChRα211/D14A/W149P/V155K, AChRα211/D14A/W149P/V155A, AChRα211/D14A/W149P/V155M, AChRα211/D14A/W149P/V155D, AChRα211/D14A/W149P/V155P, and AChRα211/D14A/W149P/V155G; any one transmembrane region selected from a CD8α transmembrane domain (SEQ ID NO: 6), a CD28 transmembrane domain (SEQ ID NO: 37), and an AChRα transmembrane domain (SEQ ID NO: 38); any one co-stimulatory domain selected from a 4-1BB co-stimulatory domain (SEQ ID NO: 7), a CD28 co-stimulatory domain (SEQ ID NO: 25), a GITR co-stimulatory domain (SEQ ID NO: 26), a TNFR2 co-stimulatory domain (SEQ ID NO: 27), a DR3 co-stimulatory domain (SEQ ID NO: 28), a CD30 co-stimulatory domain (SEQ ID NO: 29), an HVEM co-stimulatory domain (SEQ ID NO: 30), a CD27 co-stimulatory domain (SEQ ID NO: 31), and an OX40 co-stimulatory domain (SEQ ID NO: 32); and an intracellular signaling domain which is a CD3ζ intracellular signaling domain (SEQ ID NO: 8); and optionally comprising a linker region (which is a partial or whole sequence of a CD8α hinge sequence (SEQ ID NO: 39), a CD28 hinge sequence (SEQ ID NO: 40), or an artificial linker sequence (SEQ ID NO: 41)) between the antigenic region and the transmembrane region.

In one aspect, the chimeric receptor of the present invention can be a polypeptide that comprises an amino acid sequence having 80% or higher (preferably 85% or higher, 900 or higher, 950 or higher, 960 or higher, 970 or higher, 980 or higher, or 990 or higher) sequence identity to an amino acid sequence of any of SEQ ID NOs: 43 to 47, and retains a function as a CAAR according to the present invention. Such a polypeptide may comprise, at its N-terminus, a signal peptide for enabling expression on the cell surface, and a tag peptide or the like for confirming expression on the cell surface may further be linked to the N-terminus of the amino acid sequence of any of SEQ ID NOs: 43 to 47, either directly or indirectly via a spacer. The chimeric receptor may be a polypeptide consisting of an amino acid sequence derived from the amino acid sequence described above by incorporating the substitution, deletion, insertion, and/or addition of several (20 or fewer, preferably 15 or fewer, 10, 9, 8, 7, 6, 5, 4, 3, 2, or 1) amino acids. In the amino acid sequence of the polypeptide, preferably, amino acids at positions 1 to 160 (preferably positions 1 to 170, more preferably positions 1 to 180, positions 1 to 190, positions 1 to 200, or positions 1 to 211) in SEQ ID NOs: 43 to 47 are maintained, and amino acids in the other positions may be changed at a certain level of sequence identity. Such an amino acid sequence of the chimeric receptor is most preferably any one amino acid sequence of SEQ ID NOs: 43 to 47.

### <Gene>

The present invention provides a polynucleotide comprising a region encoding the chimeric receptor polypeptide described above. The polynucleotide of the present invention may be a nucleotide fragment, mRNA, or the like comprising the region and may be in a form, such as a plasmid, a vector, or a virus, suitable for gene transfer and enabling the chimeric receptor to be expressed inthe cell membrane.

The polynucleotide of the present invention may be DNA or RNA having a native structure or may be a modified nucleic acid having a chemically modified structure. For example, when the polynucleotide of the present invention is provided in the form of mRNA to cells, a modified nucleic acid is used in order to render the mRNA resistant to degradation by RNase. Preferably, a base moiety of the mRNA is modified.. For example, a pyrimidine nucleotide having a substitution at the 5-position or pseudouridine having a substitution at the 1-position is preferred. Specific examples thereof can include 5-methylcytidine, 5-methoxyuridine, 5-methyluridine, pseudouridine, and 1-alkylpseudouridine. The 1-alkylpseudouridine may be 1-(C1-C6 alkyl)pseudouridine and is preferably 1-methylpseudouridine or 1-ethylpseudouridine.

The polynucleotide encoding the chimeric receptor of the present invention can be prepared easily from a disclosed amino acid sequence by routine methods. A nucleotide sequence encoding the amino acid sequence can be obtained on the basis of an amino acid sequence as set forth in the sequence listing. The nucleotide sequence can be obtained from NCBI RefSeq ID numbers or GenBank accession numbers mentioned above corresponding to the amino acid sequence of each region, and the polynucleotide of the present invention can be prepared using standard molecular biological and/or chemical procedures. For example, a nucleic acid can be synthesized on the basis of the nucleotide sequence, and the polynucleotide of the present invention can be prepared by combining DNA fragments obtained from a cDNA library through polymerase chain reaction (PCR).

The polynucleotide of the present invention can be linked to another nucleic acid so as to be expressed under the control of a preferred promoter. Examples of the promoter include constitutive promoters that continuously promote the expression of a gene or an operably linked construct, and inducible promoters that induce the expression of a gene or an operably linked construct by the action of a drug or the like (e.g., tetracycline or doxorubicin). The polynucleotide of the present disclosure can be also linked to other regulatory elements, for example, an enhancer sequence or a terminator sequence, which cooperate with a promoter or a transcription initiation site, in order to obtain the efficient transcription of the nucleic acid. The polynucleotide of the present invention may be associated with a gene capable of serving as a marker for confirming expression of the polynucleotide (e.g., a drug resistance gene, a gene encoding a reporter enzyme, or a gene encoding a fluorescent protein such as GFP or BFP), a tag sequence (FLAG, His tag, etc.).

The polynucleotide of the present invention may have a codon-optimized nucleotide sequence for expression in particular host cells. The nucleotide sequence thus optimized can be obtained from the amino acid sequence of interest by the application of an algorithm or software known in the art.

The present invention provides a composition comprising an effective amount of the polynucleotide of the present invention together with a pharmaceutically acceptable excipient. Preferred pharmaceutically acceptable excipients are well known to those skilled in the art. Examples of the pharmaceutically acceptable excipient include phosphate buffered saline (e.g., 0.01 M phosphate, 0.138 M NaCl, 0.0027 M KCl, pH 7.4), aqueous solutions containing inorganic acid salts such as hydrochloride, hydrobromide, phosphate, or sulfate, saline, solutions of glycol or ethanol, and salts of organic acids such as acetate, propionate, malonate and benzoate. Adjuvants such as a wetting agent or an emulsifier, and a pH buffering agent may be used. Excipients described in Remington's Pharmaceutical Sciences (Mack Pub. Co., N.J. 1991) (which is incorporated herein by reference) can be appropriately used as the pharmaceutically acceptable excipient. The composition of the present disclosure can be formulated into a known form preferred for parenteral administration, for example, injection or infusion. Further, the composition of the present disclosure may contain formulation additives such as a suspending agent, a preservative, a stabilizer and/or a dispersant, and a preservative for prolonging efficacy during preservation. The composition may be in a dry form for reconstitution with an appropriate sterile liquid before use. For administration mediated by fine particles, particles such as gold particles of a microscopic size can be coated with DNA.

In the case of transferring the polynucleotide of the present invention to cells ex *vivo,* the polynucleotide of the present invention may be combined with a substance that promotes its transfer to cells, for example, a reagent for nucleic acid transfer, such as a liposome or a cationic lipid, in addition to the excipient mentioned above. Alternatively, a vector comprising the polynucleotide of the present invention is also useful, as mentioned below.

The composition containing the polynucleotide of the present invention as an active ingredient can be administered for the treatment of an autoimmune disease caused by an autoantibody against nAChRα1 (particularly, myasthenia gravis). The composition comprising the polynucleotide of the present invention as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, or can be appropriately formulated for such administration, though the administration route is not particularly limited.

The vector comprising the polynucleotide of the present invention is not particularly limited as long as the vector is generally used in the biogenetic field. A plasmid vector, a virus vector, a non-virus vector, or the like can be used. The present invention provides such a vector comprising the polynucleotide of the present invention.

For example, a virus vector such as a retrovirus vector (including an oncoretrovirus vector, a lentivirus vector, and a pseudotyped vector), an adenovirus vector, an adeno-associated virus (AAV) vector, a simian virus vector, a vaccinia virus vector, a Sendai virus vector, an Epstein-Barr virus (EBV) vector, or a HSV vector can be used as the virus vector in the present invention. For example, a virus vector devoid of replicating ability so as not to self-replicate in infected cells can be used.

As a further example, a liposome or fine particles comprising a cationic lipid or the like can be used as the non-virus vector, as described in WO96/10038, WO97/18185, WO97/25329, WO97/30170, and WO97/31934 (which are incorporated herein by reference). As an additional example, an outer shell which is obtained by destroying, through radiation, the inner part of virus particles has been used in a method for transferring an episomal vector for the expression of scaffold/matrix attachment regions as described in Jin et al., EMBO Mol Med. 2016 Jul; 8 (7): 702-711, and can be used in the present invention; a transposon-based transfer method such as the PiggyBac method described in Mol Ther Methods Clin Dev. 2017 Dec 22; 8: 131-140, may also be used.

For the preparation of the virus vector, a plasmid comprising a sequence encoding the chimeric receptor of the present invention and a sequence necessary for enclosure in a virus (e.g., an LTR sequence) is designed to enable appropriate expression of the polypeptide of the present invention in cells infected with the virus, and the plasmid is transferred to appropriate cells (particularly, packaging cells). A virus fraction is collected and concentrated for production of the virus vector. The present invention provides such a plasmid for use in the production of a virus vector.

The plasmid used to produce the virus vector can be prepared using a commercially available kit in accordance with the attached protocol. Various kits appropriate for the type of the virus are commercially available as such a kit. Examples of the plasmid preparation kits include pLVSIN EF1α pur (Takara Bio Inc., 6186) for lentivirus, pQCXIX (Takara Bio Inc., Z1515N) for retrovirus, and pAAV-CMV (Takara Bio Inc., 6651) for adeno-associated virus.

In one aspect, the virus which encloses the polynucleotide of the present invention can be prepared by transferring the plasmid for virus preparation described above to packaging cells harboring constituent genes necessary for virus particle formation, and collecting a solution of the cultured cells. Examples of the packaging cells that can be used include PG13 (ATCC CRL-10686), PA317 (ATCC CRL-9078), GP+E-86 and GP+envAm-12 (U.S. Patent No. 5,278,056), and Psi-Crip [Proceedings of the National Academy of Sciences of the United States of America, vol. 85, pp. 6460-6464 (1988)].

In another aspect, the virus in which the polynucleotide of the present invention is enclosed can be prepared by transferring the plasmid for virus preparation described above, together with constituent genes necessary for virus particle formation, to cells, and recovering a solution of the cultured cells. A packaging signal sequence transfer reagent can be appropriately selected depending on the type of the virus or in accordance with the protocol of the preparation kit. For lentivirus, for example, Lentiviral High Titer Packaging Mix (Takara Bio Inc., 6194) can be used. As s further example, 293 cells or 293T cells having high transfection efficiency (specifically, Lenti-X 293T Cell Line (Takara Bio Inc., 632180)) can be used. An auxiliary transfer reagent (e.g., Opti-MEM I Reduced Serum Media (Thermo Fisher Scientific Inc., 31985070)) may be added at the time of transfer of the plasmid.

The virus vector of the present invention may be provided as a supernatant of the cultured cells harboring the plasmid described above, or the virus vector may be concentrated for use. For the concentration of the virus vector, an appropriate kit (e.g., Lenti-X concentrator (Takara Bio Inc., 631232)) can be used depending on the type of the virus vector.

The plasmid of the present invention is usually stored in a form in which it is retained in appropriate cells, and can be used to produce the virus vector at a desired time by culturing the cells containing the plasmid to enable proliferation, and then applying thereto stimulation for inducing virus production. The virus vector may be obtained by separating or concentrating a virus component from the culture solution. Such plasmid-containing cells for storage are not particularly limited, and cell types which are used in techniques known in the art can be used.

To produce the virus vector, Lentivirus plasmids, Lentiviral High Titer Packaging Mix (Takara Bio Inc., 6194), and TransIT-293 Transfection Reagent (Takara Bio Inc., MIR2704) are mixed with Opti-MEM I Reduced Serum Media (Thermo Fisher Scientific Inc., 31985070), incubated for 15 minutes, and then added to Lenti-X 293T Cell Line (Takara Bio Inc., 632180) which has been cultured beforehand such that the cells are semiconfluent. The culture supernatant is collected 24 to 48 hours after addition. The virus vector contained in the culture supernatant is concentrated using Lenti-X concentrator (Takara Bio Inc., 631232) in accordance with the manufacturer's protocol.

### <Cell and method for producing same>

The present invention provides a host cell containing the polynucleotide encoding the chimeric receptor described above.

A cell derived from a mammal, for example, a human cell, or a cell derived from a non-human mammal such as a monkey, a mouse, a rat, a pig, a horse, or a dog can be used in the present invention. A human cell is preferred.

The cell used in the present disclosure is not particularly limited by its type, and any type of cell can be used. For example, a cell collected, isolated, or purified from a body fluid, a tissue or an organ, for example, blood (peripheral blood, umbilical cord blood etc.) or bone marrow, or a pluripotent stem cell, such as an iPS cell or an ES cell, which differentiates into the cell mentioned above can be used (see e.g., Themeli et al., 2013). A peripheral blood mononuclear cell (PBMC), an immune cell (including, for example, a T cell, a dendritic cell, a B cell, a hematopoietic stem cell, a macrophage, a monocyte, a NK cell or a hematopoietic cell (a neutrophil or a basophil)), an umbilical cord blood mononuclear cell, a fibroblast, a precursor adipocyte, a hepatocyte, a skin keratinocyte, a mesenchymal stem cell, an adipose stem cell, various cancer cell lines, or a neural stem cell can be used. For example, a NK cell or a T cell, a precursor cell of a T cell (a hematopoietic stem cell, a lymphocyte precursor cell etc.) or a cell population containing these cells can be used. Examples of the T cell include CD8-positive T cells, CD4-positive T cells, regulatory T cells, cytotoxic T cells, and tumor infiltrating lymphocytes. The cell population containing a T cell and a precursor cell of a T cell includes PBMCs. These cells may be collected from a living body, may be obtained by the expansion culture of cells collected from a living body, or may be established as a cell line. If transplantation of the prepared CAAR-expressing cell or a cell obtained by the differentiation of the prepared CAAR-expressing cell into a living body is desired, cells collected from the living body itself or another living body of the same species can be transfected with the polynucleotide of the present invention.

In one aspect, the genetically engineered cell of the present invention is a cell expressing the chimeric receptor described above in the cell membrane. Such a cell has properties of binding to particular pathogenic B cells via the antigenic region of the chimeric receptor, and transducing the binding signal to the intracellular signaling domain so that the cell is activated. The activation of the cell expressing the chimeric receptor of the present invention differs depending on the type of the host cell and the intracellular domain contained in the chimeric receptor, and can be determined on the basis of a parameter, for example, cytokine release, improvement in cell proliferation rate, or change in cell surface molecule.

When the host cell of the genetically engineered cells of the present invention is a T cell, such a cell is also called CAAR-T cell. When the host cell is a T cell, the cell activated by signal transduction described above releases a cytotoxic cytokine (a tumor necrosis factor, lymphotoxin, etc.) and exerts cytocidal activity or cytotoxicity against target cells. In addition, the cytokine release or the change in cell surface molecule stimulates other immunocytes, for example, B cells, dendritic cells, NK cells, and macrophages. Consequently, cells that produce an autoantibody against nAchRα1 can be decreased or eliminated from the body, and a disease or a symptom caused by the autoantibody can be treated.

A method for preparing the genetically engineered cell of the present invention comprises the step of transfecting a cell with the polynucleotide of the present invention. This step is performed ex *vivo* or *in vitro.* For example, a cell can be transfected ex *vivo* or *in vitro* in accordance with routine methods using a virus vector or a non-virus vector comprising the polynucleotide of the present invention so as to obtain a cell expressing the chimeric receptor of the present invention.

Suitable methods for transfecting a cell with the polynucleotide of the present invention are known in the art. Standard biogenetic and molecular biological approaches are typically applied to methods for preparing CAR-T cells. The polynucleotide of the present invention can be transferred to the cell of interest by using various transfection techniques or the like that are well-known in the art. In another aspect, the polynucleotide of the present invention can be transferred to the genome of the cell of interest, for example, by a genome editing technique using CRISPR/Cas9 or Zn finger nuclease (e.g., U.S. Patent No. 8,956,828).

In one aspect, a patient-derived antibody or mAb35 antibody is capable of binding to the chimeric receptor expressed on the cell surface of the genetically engineered T cell of the present invention.

In one aspect, the genetically engineered T cell of the present invention exhibits cytotoxic activity against a hybridoma that produces mAb35.

In one aspect, the present invention provides a method for preparing genetically engineered cells, comprising transfecting cells with the polynucleotide or the vector of the present invention.

When the method of the present invention is applied to autologous cell therapy, a method comprising the following steps may be adopted:
a) Collecting cells to be engineered from an individual to be treated;
b) transfecting the collected cells with a vector comprising the polynucleotide of the present invention; and
c) expansion-culturing a cell population comprising chimeric receptor-expressing cells.

In one aspect, the collected cells to be engineered (preferably a cell population comprising T cells, more preferably PBMCs or a CD3-positive cell fraction) are stimulated with a soluble or membrane-bound anti-CD3 antibody (e.g., OKT3 or mOKT3) and/or APC (e.g., artificial APC (aAPC) or APC expressing a membrane-type anti-CD3 monoclonal antibody) before transfection.

In another aspect, the cells to be engineered for use in transfection are suspended at a given cell density (e.g., 0.1 to 2 × 10⁶ cells/mL) and added to a virus-bound plate. The virus-bound plate can be prepared by adding the virus concentrate described above to a plate coated with 5 to 10 ng/mL anti-CD3 antibody (clone name: OKT3) and 20 to 100 µg/mL RetroNectin.

For example, a medium supplemented with serum or a serum replacement and a cytokine such as IL-2 (e.g., AIM-V medium (Thermo Fisher Scientific Inc., 12055083) can be used as a medium for transfection. The virus-bound plate supplemented with the cells may be centrifuged.

The transfection step b) may be repeated a plurality of times. For example, a transduction step can be performed until a sufficient expression level is achieved. As a further example, the transduction step can be performed 2 to 10 times, preferably 2, 3, 4, or 5 times.

In one aspect, in the transfection step, the cells may be continuously cultured for two or more consecutive days, for example, two consecutive days, three consecutive days, or four consecutive days. In the case of using a virus-bound plate, the medium is replaced with a fresh one every 2 to 3 days, and the cells may be cultured for 5 to 20 consecutive days.

In one aspect, the genetically engineered cells of the present invention are stimulated with an anti-nAChRα1 antibody or irradiated cells expressing the BCR that recognizes nAChRα1. For example, the genetically engineered T cells of the present invention are stimulated with irradiated cells at an effector:target ratio of 100:1, 75:1, 50:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:50, or 1:100.

When the genetically engineered cells of the present invention are used in allogeneic cell therapy, the polynucleotide of the present invention can be transferred to cells (e.g., pluripotent stem cells such as ES cells or iPS cells, hemocytic cells obtained by inducing differentiation of these cells, or somatic cells or hemocytic cells which have been treated so as to circumvent immune rejection) by a usual transfection method or a gene editing method. The polynucleotide of the present invention is transferred, for example, to the genomes of pluripotent stem cells such as ES cells or iPS cells by a genome editing technique to establish pluripotent stem cells with the polynucleotide appropriately integrated. Since the pluripotent stem cells are capable of proliferating, the genetically engineered cells of the present invention can be used in allogeneic cell therapy by inducing a necessary amount of proliferation at the required time, and inducing differentiation into T cells.

### <Pharmaceutical composition and treatment method>

The genetically engineered cells of the present invention can be used in the treatment of an autoimmune disease in a subject caused by an autoantibody against nAchRα1. The treatment with the genetically engineered cells of the present invention is achieved by decreasing the number of pathogenic B cells expressing an anti-nAChRα1 autoantibody in the subject. The present invention provides, for example, a therapeutic drug for an autoimmune disease containing such genetically engineered cells as an active ingredient, and a method for treating an autoimmune disease by administering the cells. Typical examples of autoimmune diseases include myasthenia gravis (MG).

The effective amount of the CAAR-T cells as referred to in the present specification means an amount that brings about a desirable therapeutic endpoint (e.g., reduction, improvement, or removal of a symptom, or reduction or removal of a human patient's autoantibody or conspecific antibody) without bringing about toxicity to a human patient. As a mere example, the effective amount of the CAAR-T cells to be administered to a human patient may mean approximately 10⁴ to approximately 10⁹ CAAR-T cells (e.g., approximately 10⁵ to approximately 10⁶ CAAR-T cells) per kg of body weight of the human patient.

The therapeutic drug containing the cells of the present invention as an active ingredient can be administered intradermally, intramuscularly, subcutaneously, intraperitoneally, intranasally, intraarterially, intravenously, intratumorally, or into an afferent lymph vessel by parenteral administration, for example, by injection or infusion, though the administration route is not limited. Intravenous administration is preferred.

The patient to be treated with the cells of the present invention may be subjected to various pretreatments known in the field of CAR-T cells.

The binding of an anti-nAChRα1 autoantibody to the cells of the present invention in the body of a patient might reduce their pharmacological effect because cells recognized by the autoantibody undergo elimination by macrophages in the body. Hence, the patient to be treated with the cells of the present invention may be pretreated so as to transiently attain, before administration, a state of having a decreased amount of an anti-nAChRα1 autoantibody in the blood or an increased amount of IgG in the blood, and the cells of the present invention can be administered in an environment where the cells of the present invention can bind to pathogenic B cells. The cells of the present invention may be administered to a patient after dialysis as an example of such a pretreatment. The patient undergoes dialysis so that a plasma component in blood is removed to gradually decrease the level of an anti-AChRα1 antibody in the blood.

Further, the definitions and embodiments described in particular sections are intended to be applicable to other embodiments herein described for which they are suitable as would be understood by those skilled in the art. For example, in the following passages, different aspects of the present invention are defined in more detail. Each aspect thus defined may be combined with any other aspect or aspects unless clearly indicated to the contrary. In particular, any feature indicated as being preferred or advantageous may be combined with any other feature or features indicated as being preferred or advantageous.

The above disclosure generally describes the present application. A more complete understanding can be obtained by reference to Examples given below. These examples are described merely for the purpose of illustration and are not intended to limit the scope of the present application. Change in form and substitution of equivalents are also contemplated as circumstances might suggest or render expedient. Although particular terms are used in the present specification, such terms are intended in a descriptive sense and not for purposes of limitation.

### Examples

In the references to an AchRα211 mutant (SEQ ID NO: 3) used in the Examples provided below, amino acids represented by Xaa in SEQ ID NO: 3 refer to the same amino acids at the corresponding sites in the original sequence of SEQ ID NO: 2 unless particular amino acids are specified (e.g., Xaa8 is V, Xaa14 is D, Xaa70 is D, Xaa72 is Y, Xaa112 is Y, Xaa192 is C, and Xaa193 is C). In the reference to an AchRα236 mutant (SEQ ID NO: 4), amino acids represented by Xaa in SEQ ID NO: 3 refer to the same amino acids at the corresponding sites in the original sequence of SEQ ID NO: 1 unless particular amino acids are specified.

In the description of chimeric receptors comprising an extracellular region of each AchRα as an antigenic region (AchRα-CAAR) in the Examples provided below, human-derived sequences are used, and a CD8α-derived linker region (SEQ ID NO: 5) as a linker region, a CD8α-derived transmembrane domain (SEQ ID NO: 6) as a transmembrane region, a 4-1BB-derived co-stimulatory domain (SEQ ID NO: 7) as an intracellular co-stimulatory domain, and a CD3ζ-derived intracellular signal domain (SEQ ID NO: 8) as an intracellular signal domain are used, unless otherwise specified. Also, a CD8α signal peptide (SEQ ID NO: 9) is used as a signal peptide. For the confirmation of CAAR expression, a FLAG tag sequence is placed via a 3-amino acid spacer (GSG) at the N-terminus of AchRα-CAAR. For the confirmation of gene transfer, a self-cleaving peptide T2A and a fluorescent protein BFP are placed via a 3-amino acid spacer (GSG) at the C-terminus of AchRα-CAAR.

In Examples provided below, the following methods were adopted as respective experimental approaches.

### [Construction of virus vector for CAAR expression]

A lentivirus vector was constructed for expressing AchRα-CAAR (Figure 1) in which an antigenic region, a linker region, a transmembrane region, an intracellular co-stimulatory domain, and an intracellular signal region were arranged in that order from the N-terminus. Also, a signal peptide was placed at the N-terminus of AchRα-CAAR. In order to confirm expression, etc., a FLAG tag sequence and a 3-amino acid spacer (GSG) were placed at the N-terminus of AchRα-CAAR, whilst a self-cleaving peptide T2A and a fluorescent protein BFP were placed at the C-terminus thereof. A nucleotide sequence corresponding to the arrangement as described above was inserted to a multiple cloning site of pLVSIN EF1α pur (Takara Bio Inc., 6186) to prepare a plasmid vector for insertion into lentivirus in order to express AchRα-CAAR.

### [Preparation of AchRα CAAR-T cell]

In accordance with the protocol of pLVSIN EF1α pur, the plasmid vector for lentivirus, Lentiviral High Titer Packaging Mix (Takara Bio Inc., 6194), and TransIT-293 Transfection Reagent (Takara Bio Inc., MIR2704) were mixed with Opti-MEM I Reduced Serum Media (Thermo Fisher Scientific Inc., 31985070), incubated for 15 minutes, and then added to Lenti-X 293T Cell Line (Takara Bio Inc., 632180). Cells of the Lenti-X 293T Cell Line had been cultured before until they were semi-confluent. On the next day, the medium was replaced with a fresh one, and the cells were further cultured for 24 hours, followed by the recovery of a supernatant. A virus contained in the culture supernatant was concentrated using Lenti-X concentrator (Takara Bio Inc., 631232) in accordance with the manufacturer's protocol. The virus concentrate was added to a plate coated with 5 to 10 ng/mL anti-CD3 antibody (clone name: OKT3) and 20 to 100 µg/mL RetroNectin in accordance with the manufacturer's protocol (Takara Bio Inc., T100B) to prepare a virus-bound plate. Next, human-derived peripheral blood mononuclear cells were suspended into a medium (hereinafter, referred to as a growth medium) obtained by adding 100 U/mL IL-2 to AIM-V medium (Thermo Fisher Scientific Inc., 12055083) containing 5% fetal bovine serum (FBS) (hereinafter, referred to as a basal medium), at a concentration of 0.1 to 2 × 10⁶ cells/mL. The suspension was added to the virus-bound plate, centrifuged at 1000 g for 30 to 60 minutes, and then transferred to an incubator, and cultured. The medium was replaced with a fresh growth medium every two to three days, and the cells were cultured for 7 to 14 days to prepare T cells expressing AchRα-CAAR (AchRαCAAR-T). If necessary, the recovered cells were cryopreserved using CELLBANKER 1 (Takara Bio Inc., CB011). The frozen cells, when used in an experiment, were used after being thawed and then incubated in a medium for 1 day.

### [Method for confirming expression of CAAR in cells]

An anti-AchRα antibody (mAb35, prepared from an ATCC-derived hybridoma (TIB-175)) was suspended into 1 to 10 µg/mL in a FACS buffer (phosphate buffered saline containing 0.5% BSA, 2 mM EDTA, and 0.09% azide), then added to the cells, and reacted at 4°C for 10 to 20 minutes, and the cells were washed with a FACS buffer. APC anti-human IgG Fc Antibody (BioLegend, Inc., 409306) was diluted 20- to 100-fold with a FACS Buffer, then added to the cells, and reacted at 4°C for 10 to 20 minutes, and the cells were washed with a FACS buffer. Depending on a test, APC anti-FLAG tag antibody (BioLegend, Inc., 637308) was diluted 20- to 100-fold with a FACS Buffer, then added to the cells, and reacted at 4°C for 10 to 20 minutes, and the cells were washed with a FACS buffer. The antibody-stained cells were suspended in a FACS buffer containing 1 to 10 µg/mL 7-aminoactinomycin D and measured by use of flow cytometry (MACSQUANT Analyzer 10). After output as an FCS file, a cell fraction was selected in an FSC/SSC plot using FLOW JO software to select live cells which were negative to 7-aminoactinomycin D staining. Next, the live cells were gated with BFP on the X axis against APC on the Y axis, and indicated by quartering lines using the fluorescence of APC in BFP-negative cells as a negative control. If necessary, the live cells were fractionated into BFP-positive cells or BFP-negative cells, and these groups were overlaid and gated with APC on the X axis.

### <Example 1: Study on antigenic region capable of being expressed as CAAR in cell membranes>

In order to find a CAAR having an appropriate structure (Figure 1) for use in the treatment of myasthenia gravis, a virus vector for CAAR expression was prepared by using sequences given below as a signal peptide, antigenic region, linker region, and transmembrane domain; and the expression of CAAR in a cell membrane was evaluated. In this experiment, a 4-1BB was used as an intracellular signal domain (SEQ ID NO: 7), and CD3ζ (SEQ ID NO: 8) was used as an intracellular signal domain.
(1) Lentivirus prepared using a vector for CAAR comprising a native AchRα extracellular region (SEQ ID NO: 1) as an antigenic region, a CD8α-derived linker region (SEQ ID NO: 5), and a CD8α-derived transmembrane domain (SEQ ID NO: 6) arranged, the CAAR being linked to a CD8α-derived signal peptide (SEQ ID NO: 9) (Figure 2A).
(2) Lentivirus prepared using a vector for CAAR comprising a native AchRα extracellular region(SEQ ID NO: 1) as an antigenic region, a CD8α-derived linker region (SEQ ID NO: 5), and an AchRα-derived transmembrane domain (SEQ ID NO: 38) arranged, the CAAR being linked to a CD8α-derived signal peptide (SEQ ID NO: 9) (Figure 2A).
(3) Lentivirus prepared using a vector for CAAR comprising a native AchRα extracellular region (SEQ ID NO: 1) as an antigenic region, a CD8α-derived linker region (SEQ ID NO: 5), and a CD8α-derived transmembrane domain (SEQ ID NO: 6) arranged, the CAAR being linked to an AchRα-derived signal peptide (SEQ ID NO: 10) (Figure 2B) .
(4) Lentivirus prepared using a vector for CAAR comprising a native AchRα extracellular region (SEQ ID NO: 1) as an antigenic region, a CD8α-derived linker region (SEQ ID NO: 5), and a CD8α-derived transmembrane domain (SEQ ID NO: 6) arranged, the CAAR being linked to an immunoglobulin-derived signal peptide (SEQ ID NO: 11) (Figure 2B).
(5) Lentivirus prepared by co-expression of the vector described in (1), (3), or (4) with a vector expressing a complex of the following components: full-length AchRβ (SEQ ID NO: 12), full-length AchRδ (SEQ ID NO: 13), full-length AchRε (SEQ ID NO: 14), and GFP linked through T2A (Figure 2C). In general, if each of the components contained in a multimer is expressed alone, stable membrane expression is difficult to obtain due to an exposed binding face between the components. Since native AchR is expressed as a pentamer, it has been reported that, particularly, fetal AchR (α, α, β, δ, and ε) has a high expression level in cell lines (Lozier et al., Am J Clin Pathol. 2015 Feb; 143 (2): 186-92; quiz 305) .
(6) Lentivirus prepared using a vector for CAAR for use in the development of disease models with myasthenia gravis comprising a main immunogenic region (SEQ ID NO: 15) as an antigenic region, a CD8α-derived linker region (SEQ ID NO: 5), and a CD8α-derived transmembrane domain (SEQ ID NO: 6) arranged, the CAAR being linked to a CD8α-derived signal peptide (SEQ ID NO: 9) (Figure 2D).
(7) In the structural analysis of an AchRα-mAb35 antibody-bungarotoxin (α-Btx) complex, four mutations in the extracellular domain (236 amino acids) of AchRα (Noridomi et al., Elife. 2017 Apr 25; 6: e23043) (Δ59-83, V8E, W174R, and V180A) are disclosed for the purpose of crystallizing the protein prepared from yeast. Lentivirus prepared using a vector for CAAR comprising a sequence containing three (V8E/W174R/V180A) of these mutations as an antigenic region, a CD8α-derived linker region (SEQ ID NO: 5), and a CD8α-derived transmembrane domain (SEQ ID NO: 6) arranged, the CAAR being linked to a CD8α-derived signal peptide (SEQ ID NO: 9) (Figure 2E).
(8) Lentivirus prepared using a vector for CAAR comprising a sequence containing the four mutations (Δ59-83, V8E, W174R, and V180A) described above as an antigenic region, a CD8α-derived linker region (SEQ ID NO: 5), and a CD8α-derived transmembrane domain (SEQ ID NO: 6) arranged, the CAAR being linked to a CD8α-derived signal peptide (SEQ ID NO: 9) (Figure 2E).

### [Results]

For (1) to (5) and (7), the cell membrane expression of CAAR was not confirmed in BFP-positive cells compared with BFP-negative cells. For (6), CAAR was expressed in a cell membrane. However, the CAAR lacked mAb35 antibody reactivity. Only in the case of (8) where four mutations were introduced, membrane expression of CAAR along with mAb35 antibody reactivity was confirmed in the BFP-positive cells compared with the BFP-negative cells. The paper discussing the crystal structure of AchRα mentioned in (7) examined protein expression using yeast and did not examine expression in a cell membrane using animal cells nor did the paper examine the expression of AchRα as a monomer. In this paper, crystallization with a high resolution was successfully achieved by the addition of α-Btx which bound to AchRα. However, the use of α-Btx, i.e., snake toxin, in CAAR-T therapy cannot be accepted from a safety standpoint. In conclusion, the membrane expression of AchRα-CAAR confirmed for the vector of (8) was surprising.

### [Discussion]

For (1) to (5) and (7), the cell membrane expression of CAAR could not be confirmed in BFP-positive cells compared with BFP-negative cells, suggesting that the extracellular region of native AchRα fails to undergo membrane expression as a chimeric receptor. In the results for (6), the BFP-positive cells reacted with an anti-FLAG antibody, but not with mAb35 antibody. This suggests that the mAb35 antibody does not recognize at least a linear main immunogenic region. Thus, this antibody was found unusable in the removal of pathogenic B cells.

In Examples given below relating to a mutated version of the AchRα extracellular region, the full-length sequence of the extracellular region (236 amino acids) is referred to as AchRα236, and a mutant having a Δ59-83 deletion in the sequence of AchRα236 is referred to as AchRα211. Substitution mutations of amino acids are represented by the respective original and substituent amino acids delimited by "/". Amino acid positions equal to or subsequent to position 84 are represented by the corresponding amino acid positions in the sequence of AchRα211 (W174 and V180 of AchRα236 correspond to W149 and V155, respectively, of AchRα211). Specifically, a mutant having the four mutations adopted in (8) is represented by AchRα211/V8E/W149R/V155A.

### <Example 2: Study on AchRα mutant to be applied to CAAR - 1>

In order to further improve the level of expression of CAAR in the cell membrane, a further mutation was studied in addition to the four mutations (AchRα211/V8E/W149R/V155A) provided in the vector of (8) in Example 1. α-Btx as used in the aforementioned literature to obtain the crystal structure of AchRα-mAb35 antibody-α-Btx binds to C192 and C193 of AchRα211 and stabilizes the structure of the complex, suggesting that C192 and C193 of AchRα211 are in a free form in vector (8) of Example 1, which may cause AchRα211 to be unstable when expressed in a cell membrane. Accordingly, an expression vector of AchRα-CAAR comprising AchRα211/V8E/W149R/V155A/C192G/C193G with C192 and C193 mutated to glycine as an antigenic region (antigenic region: the amino acid sequence of SEQ ID NO: 3 wherein Xaa8 is E, Xaa149 is R, Xaa155 is A, Xaa192 and Xaa193 are GG, and the other amino acids Xaa are amino acids at the corresponding positions in SEQ ID NO: 2) was constructed and evaluated for expression on a cell membrane (Figure 3).

### [Results]

It was confirmed that AchRα-CAAR comprising the additional C192G/C193G mutations exhibited the same level of membrane expression as that of AchRα211/V8E/W149R/V155A.

### <Example 3: Study on AchRα mutant to be applied to CAAR - 2>

AchRα-CAAR containing the four mutations was expressed in a cell membrane. In order to evaluate the contribution of each amino acid mutation to membrane expression, a vector for expressing mutant AchRα-CAAR containing various combinations of the four mutations shown in the table below (antigenic region: the amino acid sequences shown in the table below; the amino acid sequence of SEQ ID NO: 3 wherein Xaa8, Xaa149, and Xaa155 are amino acids defined in the table below, and the other amino acids Xaa are amino acids at the corresponding positions in SEQ ID NO: 2; and the amino acid sequence of SEQ ID NO: 4 wherein Xaa8, Xaa174, and Xaa180 are amino acids defined in the table below, and the other amino acids Xaa are amino acids at the corresponding positions in SEQ ID NO: 1) was constructed and evaluated for expression in a cell membrane (Figure 4).

**[Table 1]**

| Name | Antigenic region | Xaa8 | Xaa149 (Xaa174) | Xaa155 (Xaa180) |
|---|---|---|---|---|
| AchRα236 | SEQ ID NO: 1 | - | - | - |
| AchRα236/V8E | SEQ ID NO: 4 | E | W | V |
| AchRα236/W149R | SEQ ID NO: 4 | V | R | V |
| AchRα236/V155A | SEQ ID NO: 4 | V | W | A |
| AchRα236/V8E/W149R | SEQ ID NO: 4 | E | R | V |
| AchRα236/V8E/X155A | SEQ ID NO: 4 | E | W | A |
| AchRα236/W149R/V155A | SEQ ID NO: 4 | V | R | A |
| AchRα236/V8E/W149R/V155A | SEQ ID NO: 4 | E | R | A |
| AchRα211 | SEQ ID NO: 2 | - | - | - |
| AchRα211/V8E | SEQ ID NO: 3 | E | W | V |
| AchRα211/W149R | SEQ ID NO: 3 | V | R | V |
| AchRα211/V155A | SEQ ID NO: 3 | V | W | A |
| AchRα211/V8E/W149R | SEQ ID NO: 3 | E | R | V |
| AchRα211/V8E/X155A | SEQ ID NO: 3 | E | W | A |
| AchRα211/W149R/V155A | SEQ ID NO: 3 | V | R | A |
| AchRα211/V8E/W149R/V155A | SEQ ID NO: 3 | E | R | A |

### [Results]

The Δ59-83 mutation was found to be necessary for the expression of AchRα-CAAR in a cell membrane. Unexpectedly, a V8E mutation reduced membrane expression, whereas W149R and V155A mutations were found to markedly increase the level of membrane expression.

### <Example 4: Study on AchRα mutant to be applied to CAAR - 3>

The mutations of W149 and V155 in AchRα211 were also effective in increasing membrane expression. The substituted amino acids at these sites had enhanced hydrophilicity relative to the native hydrophobic amino acids, suggesting that the substitution of these native amino acids and native amino acids at their neighboring sites with lysine is effective for membrane expression. Accordingly, it was determined whether a similar effect could be obtained by substituting amino acids within the 146-159 region of AchRα211, which is a region rich in hydrophobic amino acids, with lysine. An expression vector of AchRα-CAAR in which each of amino acids 147, 149, 151, 153, 155, 157, and 159 in the 146-159 region of AchRα211 was substituted with lysine as a representative amino acid (antigenic region: the amino acid sequence of SEQ ID NO: 3 wherein Xaa146 to Xaa159 are a sequence shown in Table 2 below, and the other amino acids Xaa are amino acids at the corresponding positions in SEQ ID NO: 2) was constructed and evaluated for expression in a cell membrane (Figure 5).

**[Table 2]**

| Name | Xaa146-159 |
|---|---|
| AchRα211/G147K | LKTWTYDGSVVAIN (SEQ ID NO: 16) |
| AchRα211/W149K | LGTKTYDGSVVAIN (SEQ ID NO: 17) |
| AchRα211/Y151K | LGTWTKDGSVVAIN (SEQ ID NO: 18) |
| AchRα211/G153K | LGTWTYDKSVVAIN (SEQ ID NO: 19) |
| AchRα211/V155K | LGTWTYDGSKVAIN (SEQ ID NO: 20) |
| AchRα211/A157K | LGTWTYDGSVVKIN (SEQ ID NO: 21) |
| AchRα211/N159K | LGTWTYDGSVVAIK (SEQ ID NO: 22) |

### [Results]

It was confirmed that all the above AchRα-CAAR mutants were expressed in the cell membrane. Particularly, substitutions of amino acids G147, W149, V155, A157, or N159 increased the level of expression.

### <Example 5: Study of AchRα mutants to be used in AchRα-CAAR - 4>

In order to optimize the amino acid substitution of W149 and V155 in AchRα211, a mutant AchRα-CAAR expression vector shown in Table 3 below (antigenic region: the amino acid sequence of SEQ ID NO: 3 wherein Xaa149 and Xaa155 are a sequence shown in the table below, and the other amino acids Xaa are amino acids at the corresponding positions in SEQ ID NO: 2) was constructed and evaluated for expression in a cell membrane (Figures 6 and 7).

**[Table 3]**

| Name | Xaa149 | Xaa155 |
|---|---|---|
| AchRα211 | W | V |
| AchRα211/W149A | A | V |
| AchRα211/W149K | K | V |
| AchRα211/W149D | D | V |
| AchRα211/W149S | S | V |
| AchRα211/W149G | G | V |
| AchRα2111W149N | N | V |
| AchRα211/W149l | I | V |
| AchRα211/W149F | F | V |
| AchRα211/W149M | M | V |
| AchRα211/W149P | P | V |
| AchRα2111W149R | R | V |
| AchRα211/V155R | W | R |
| AchRα211/V155K | W | K |
| AchRα211A/155D | W | D |
| AchRα211A/155S | W | S |
| AchRα211/V155G | W | G |
| AchRα211/V155N | W | N |
| AchRα211/V1551 | W | I |
| AchRα211/V155F | W | F |
| AchRα211/V155M | W | M |
| AchRα211/V155P | W | P |
| AchRα211/V155A | W | A |
| AchRα211/W149R/V155K | R | K |

### [Results]

It was confirmed that all the above AchRα-CAAR mutants were expressed in a cell membrane. Particularly, the substitution of W149 with amino acids R, K, or P and the substitution of V155 with amino acids K, A, M, D, P, or G increased the level of expression. The incorporation of double mutations W149R and V155K was found to increase the level of expression by approximately 10-fold as compared to the each of the single mutations.

### <Example 6: Evaluation of cytocidal activity of AchRα-CAAR-T cell against anti-AchRα antibody-producing hybridoma>

CAAR-T cells expressing AchRα-CAAR in which mutant AchRα confirmed to be expressed in a cell membrane in the Examples described above, was provided as an antigenic region, were studied to assess their cytocidal activity against anti-AchRα antibody-producing hybridomas serving as target cells, and proliferative performance during the reaction (Figure 8).

### (1) Preparation of cell

CAAR-T cells having AchRα211, AchRα211/V8E/W149R/V155A, AchRα211/W149R, AchRα211/V155K, or AchRα211/W149R/V155K as an antigenic region were prepared in accordance with each Example described above, and suspended in a basal medium. The target cells used were hybridomas (ATCC, TIB-175) that produced the anti-AchRα antibody mAb35. In order to detect viability of the target cells, lentivirus having an insert of GFP gene was prepared by a method similar to CAAR transfection, and allowed to infect the mAb35-producing hybridomas, which were then subcloned by the limiting dilution method to establish a hybridoma cell line harboring the GFP gene. ClonaCell-HY Medium E (STEMCELL Technologies Inc., 3805) was used in hybridoma culture.

### (2) Evaluation of cytocidal activity

A BFP-positive cell count (CAAR-T cells) was determined by flow cytometry. The CAAR-T cells of each type were added at a concentration of 0.25 × 10³ to 2 × 10⁴ cells/well to a 96-well plate, while the GFP-positive hybridomas were added at a concentration of 1 × 10⁴ cells/well thereto. A basal medium and a half medium of ClonaCell-HY Medium E were used in culture. Three days later, the cells were suspended in a FACS buffer containing 1 to 10 µg/mL 7-aminoactinomycin D and analysed using flow cytometry. After output as an FCS file, a cell fraction was selected in an FSC/SSC plot using FLOW JO software to select live cells which were negative to 7-aminoactinomycin D staining. Next, the live cells were gated with BFP on the X axis against GFP on the Y axis. BFP-positive cells were selected as the CAAR-T cells, while a GFP-positive cells were selected as the target cells. The numbers of live BFP-positive cells and GFP-positive cells were determined. The cytocidal activity was assessed by determining the amount of viable target cells. The target cell count in a well without the addition of the CAAR-T cells was defined as 100%. The proliferation of the CAAR-T cells was also assessed, and the target cell count before the start of the reaction was defined as 100%.

### [Results]

All the mutant CAAR-T cells exhibited increased cytocidal activity as compared to the cytocidal activity of negative control T cells. The CAAR-T cell counts of all the mutants were maintained at the completion of the reactions. In particular, the CAAR-T cells having AChRα211/V155K or AChRα211/V149R/V155K had high proliferative performance.

### <Example 7: Improvement in cytocidal activity by structural engineering of CAAR>

As a result of the studies in Examples 1 to 6, AChRα211/W149R/V155K was set as a representative antigenic region. It has been reported that the affinity of CAR structures comprising an scFv for an antigen expressed on target cells influences the efficacy of CAR-T cells in the field of cancer therapy (Benmebarek et al., Int J Mol Sci. 2019 Mar; 20 (6): 1283). According to structure analysis using the mAb35 antibody and AChRα (Noridomi et al., Elife. 2017 Apr 25; 6: e23043), amino acids D14, D70, Y72, and Y112 of AChRα are required for the interaction of AChRα with the mAb35 antibody. In order to potentiate the cytocidal activity of CAAR-T by reducing the affinity of the antigenic region AChRα for anti-mAb35 antibody, the distance between AChRα and anti-mAb35 antibody was optimized. A virus vector for enabling expression of CAAR having a mutation introduced at a site involved in the antibody binding of AChRα211/W149R/V155K (antigenic region: the amino acid sequence of SEQ ID NO: 3 wherein Xaa14, Xaa70, Xaa72, and Xaa112 are amino acids shown in the table below, Xaa146 to Xaa159 are the amino acid sequence of SEQ ID NO: 23, and the other amino acids Xaa are amino acids at the corresponding positions in SEQ ID NO: 2) was constructed, and CAAR-T cells were prepared and evaluated for their cytocidal activity (Figure 9). The cytocidal activity evaluation was carried out by a method given below.

**[Table 4]**

| Name | Xaa14 | Xaa70 | Xaa72 | Xaa112 |
|---|---|---|---|---|
| AChRα211/W149R/V155K | D | D | Y | Y |
| AChRα211/D14A/W149RN155K | A | D | Y | Y |
| AChRα211/D14G/W149R/V155K | G | D | Y | Y |
| AChRα211/D70N/W149R/V155K | D | N | Y | Y |
| AChRα211/D70A/W149R/V155K | D | A | Y | Y |
| AChRα211/Y72F/W149R/V155K | D | D | F | Y |
| AChRα211/Y112F/W149R/V155K | D | D | Y | F |

### (1) Establishment of target cell (membrane-type mAb35-scFv-expressing RAJI cell)

A gene encoding the amino acid sequence (SEQ ID NO: 24) of mAb35-derived scFv linked to a CD8α-derived signal secretion sequence, linker region, and transmembrane region was inserted to pLVSIN EF1α IRES-ZsGreen1 (Takara Bio Inc., 6192). Lentivirus having an insert of this gene was prepared by a method similar to CAAR transfection using the vector, and allowed to infect K562 cells (ATCC, CCL-243) and RAJI cells (ATCC, CCL-86), which were then subcloned by the limiting dilution method to establish respective cell lines expressing membrane-type mAb35 antibody. RPMI1640 medium containing 10% FBS (Thermo Fisher Scientific Inc., 61870-036) was used in the culture of the established cell lines.

### (2) Evaluation of cytocidal activity

A pathogenic anti-AChRα antibody is present in blood in the body of a myasthenia gravis patient. In order for administered CAAR-T cells to exhibit a pharmacological effect, AChRα-CAAR needs to bind to the BCR expressed on pathogenic B cells or the like. The anti-AChRα antibody in blood may bind to CAAR and thereby competitively inhibit the binding to BCRs. Tonic signaling in which a sustained stimulus is delivered to a CAR structure is known to be responsible for the exhaustion of administered cells (Non Patent Literature: Long et al., Nat Med. 2015 Jun; 21 (6): 581-90. doi: 10.1038/nm.3838.). Accordingly, the cytocidal activity was evaluated in the presence or absence of the mAb35 antibody, an anti-AChRα antibody.

The FLAG-positive ratio (CAAR expression ratio) of the prepared CAAR-T cells was measured by use of flow cytometry, and a CAAR-T cell count was determined. The CAAR-T cells and the membrane-type mAb35-scFv-expressing K562 cells were suspended into a growth medium and inoculated into a 6-well plate at a concentration of 7 × 10⁵ cells /well, and cultured for 7 days. The medium was replaced with a fresh basal medium every two to three days. The culture solution was sampled and subjected to flow cytometry to confirm whether the membrane-type mAb35-scFv-expressing K562 cells detected as ZsGreen1-positive cells no longer survived. Then, a FLAG-positive cell count was conducted todetermined the number of CAAR-T cells. Next, the membrane-type mAb35-scFv-expressing RAJI cells were inoculated into a 96-well plate at a concentration of 1 × 10⁴ cells/well, while the CAAR-T cells were inoculated thereto at a concentration of 1 × 10³, 1 × 10⁴, or 2 × 10⁴ cells/well , using RPMI1640 medium containing 10% FBS. mAb35 antibody (ATCC, TIB-175-derived) was added at 10 µg/mL (final concentration) to some wells of the plate. On the next day, a FACS buffer containing 1 to 10 µg/mL 7-aminoactinomycin D was added in equal amounts thereto, followed by analysis using flow cytometry. After output as an FCS file, a cell fraction was selected in an FSC/SSC plot using FLOW JO software to select live cells which were negative to 7-aminoactinomycin D staining. Next, the live cells were gated with BFP on the X axis against ZsGreen1 on the Y axis. BFP-positive cells were selected as the CAAR-T cells, while ZsGreen1-positive cells were selected as the target cells. The numbers of live BFP-positive cells and GFP-positive cells were determined. The cytocidal activity was assessed by determining the number of viable target cells. The target cell count in a well without the addition of the CAAR-T cells was defined as 100%.

### [Results]

All the CAAR-T cells having mutant AChRα-CAAR were found to have cytocidal activity, regardless of the presence or absence of the mAb35 antibody.

### <Example 8: Improvement in cytocidal activity by tuning the structure of CAAR - 1>

A linker region is known to play an important role in the activity of CAR-T cells having scFv as a binding domain in the field of cancer. Accordingly, a virus vector for AChRα-CAARs using AChRα211/W149R/V155K as an antigenic region and using a N-terminally truncated linker region of a CD8α-derived linker region (SEQ ID NO: 5) or an artificially designed flexible linker (SEQ ID NO: 41) (antigenic region: AChRα211/W149R/V155K (the amino acid sequence of SEQ ID NO: 3 wherein Xaa146 to Xaa159 are the amino acid sequence of SEQ ID NO: 23, and the other amino acids Xaa are amino acids at the corresponding positions in SEQ ID NO: 2), linker region: a sequence shown in the table below)) was constructed, and CAAR-T cells were prepared and evaluated for their cytocidal activity by the method of Example 7 (Figure 10).

**[Table 5]**

| Name | Sequence |
|---|---|
| CD8(45) | TTTPAPRPPTPAPTIASQPLSLRPEACRPAAGGAVHTRGLDFACD (SEQ ID NO: 5) |
| CD8(19) | CRPAAGGAVHTRGLDFACD (27-45 of SEQ ID NO: 5) |
| CD8(14) | GGAVHTRGLDFACD (32-45 of SEQ ID NO: 5) |
| CD8(8) | RGLDFACD (38-45 of SEQ ID NO: 5) |
| CD8(4) | FACD (42-45 of SEQ ID NO: 5) |
| CD8(2) | CD (44-45 of SEQ ID NO: 5) |
| Flexible Linker (14) | ASGGGGSGGGGSSG (SEQ ID NO: 41) |
| Flexible Linker (8) | SGGGGSSG (7-14 of SEQ ID NO: 41) |
| Flexible Linker (4) | GSSG (11-14 of SEQ ID NO: 41) |

### [Results]

All the CAAR-T cells having mutant AChRα-CAAR were confirmed to have cytocidal activity, regardless of the presence or absence of the mAb35 antibody. The results suggested that the presence of a linker is not important for AChRα-CAAR comprising the full-length extracellular region as an antigenic region.

### <Example 9: Study of co-stimulatory domains suitable for AChRα-CAAR>

A co-stimulatory domain is known to play an important role in exerting a function of CAR-T. Accordingly, various co-stimulatory domains were studied for their effect on AChRα-CAAR by a method given below.

### (1) Preparation of CAAR-T cell

A virus vector for AChRα-CAAR comprising AChRα211/W149R/V155K as an antigenic region and FACD (42 to 45 of SEQ ID NO: 5) or FASD (42 to 45 of SEQ ID NO: 39, Xaa44 = S) as a linker region, and further comprising a co-stimulatory domain based on any of various sequences known in the art for CAR-T or various sequences known as the TNF receptor superfamily related to the longevity of T cells (antigenic region: ChRα211/W149R/V155K (the amino acid sequence of SEQ ID NO: 3 wherein Xaa146 to Xaa159 are the amino acid sequence of SEQ ID NO: 23, and the other amino acids Xaa are amino acids at the corresponding positions in SEQ ID NO: 2), co-stimulatory domain: a sequence derived from 4-1BB (SEQ ID NO: 7), CD28 (SEQ ID NO: 25), GITR (SEQ ID NO: 26), TNFR2 (SEQ ID NO: 27), DR3 (SEQ ID NO: 28), CD30 (SEQ ID NO: 29), HVEM (SEQ ID NO: 30), CD27 (SEQ ID NO: 31), or OX40 (SEQ ID NO: 32)) was constructed. Human peripheral blood mononuclear cells derived from a plurality of donors were infected with each virus vectorenabling AChRα-CAAR expression to prepare CAAR-T cells, which were then evaluated for their cytocidal activity by a method given below (Figure 11).

### (2) Establishment of target cell (membrane-type mAb35-expressing RAJI cell)

A gene encoding an amino acid sequence (SEQ ID NO: 33) corresponding to a peptide of a mAb35 antibody (rat-derived) H chain with its constant region replaced with a membrane-type human IgG1 constant region and a peptide of a mAb35 L chain with its constant region replaced with a human Igκ constant region, wherein the H chain and L chain were linked via T2A, was inserted to pLVSIN EFla IRES-ZsGreen1. Since the co-expression of CD79a and CD79b is necessary for the stable expression of a membrane-type antibody, a gene encoding the amino acid sequence (SEQ ID NO: 34) of full-length CD79a and full-length CD79b linked via T2A was inserted to pLVSIN EF1α pur. Lentivirus having an insert of each of these genes was prepared by a method similar to CAAR transfection using these vectors, and allowed to infect RAJI cells (ATCC, CCL-86), which were then subcloned by the limiting dilution method to establish a cell line expressing the membrane-type mAb35 antibody and CD79a/CD79b. RPMI1640 medium containing 10% FBS was used in the culture of the established cell line. The cell line was acclimatized by replacement with a basal medium beforehand and used in activity evaluation.

### (3) Evaluation of cytocidal activity

A BFP-positive cell count (CAAR-T cells) was determined by use of flow cytometry for each type of CAAR-T cells prepared as described above. The CAAR-T cells were inoculated into a 96-well plate at a concentration 1 × 10³ cells/well, while the target cells were inoculated thereto at a concentration of 1 × 10⁴ cells/well. The medium used was a growth medium. Two days later, additional target cells were inoculated at a concentration of 1 × 10⁵ cells/well and further cultured for 4 days. A FACS buffer containing 1 to 10 µg/mL 7-aminoactinomycin D was added in equal amounts thereto, followed by analysis using flow cytometry. After output as an FCS file, a cell fraction was selected in an FSC/SSC plot using FLOW JO software to select live cells which were negative for 7-aminoactinomycin D staining. Next, the viable cells were gated with BFP on the X axis against ZsGreen1 on the Y axis. BFP-positive cells were selected as the CAAR-T cells, while ZsGreen1-positive cells were selected as the target cells. The numbers of live BFP-positive cells and GFP-positive cells were determined. The cytocidal activity was assessed by the number of viable target cells. The target cell count in a well without the addition of the CAAR-T cells was defined as 100%.

### [Results]

The cytocidal activity of AChRα-CAARs having each of the aforementioned co-stimulatory domains was consistent amongst the donors, and AChRα-CAAR had significantly higher cytocidal activity than that of control T cells (Dunnett's-test, p < 0.01). It was thus confirmed that the AChRα-CAAR of the present invention can comprise, as a co-stimulatory domain, diverse sequences such as various co-stimulatory domains typically used in the field of CAR-T, and sequences derived from intracellular domains of various receptors belonging to the TNF receptor superfamily.

### <Example 10: Improvement in cytocidal activity by tuning of CAAR structure - 2>

In order to pursue a further study on the co-stimulatory molecule and the linker region/transmembrane region, the following study was conducted. A virus vector for each AChRα-CAAR comprising AChRα211/W149R/V155K (SEQ ID NO: 3) as an antigenic region and HVEM as a co-stimulatory domain and further comprising a linker region shown in the table below (antigenic region: AChRα211/W149R/V155K (the amino acid sequence of SEQ ID NO: 3 wherein Xaa146 to Xaa159 are the amino acid sequence of SEQ ID NO: 23, and the other amino acids Xaa are amino acids at the corresponding positions in SEQ ID NO: 2), linker region: a sequence shown in the table below, transmembrane region: a sequence shown in the table below, co-stimulatory domain: HVEM (SEQ ID NO: 30)) was constructed, and CAAR-T cells were prepared and evaluated for their cytocidal activity by the method of Example 9 using membrane-type mAb35-scFv-expressing RAJI cells (Figure 12).

**[Table 6]**

| Name | Linker region | Transmembrane domain |
|---|---|---|
| CD8(+/+45) | TTTPAPRPPTPAPTIASQPLSLRPEACRPAAGGAVHTRGLDFACD (1-45 of SEQ ID NO: 39: Xaa27=C,Xaa44=C) | SEQ ID NO: 6 |
| CD8(+/-45) | TTTPAPRPPTPAPTIASQPLSLRPEACRPAAGGAVHTRGLDFASD (1-45 of SEQ ID NO: 39: Xaa27=C, Xaa44=S) | SEQ ID NO: 6 |
| CD8(-/+45) | TTTPAPRPPTPAPTIASQPLSLRPEASRPAAGGAVHTRGLDFACD (1-45 of SEQ ID NO: 39: Xaa27=S,Xaa44=C) | SEQ ID NO: 6 |
| CD8(-/-45) | TTTPAPRPPTPAPTIASQPLSLRPEASRPAAGGAVHTRGLDFASD (1-45 of SEQ ID NO: 39: Xaa27=SXaa44=S) | SEQ ID NO: 6 |
| CD8(+/+25) | SLRPEACRPAAGGAVHTRGLDFACD (21-45 of SEQ ID NO: 39: Xaa27=C,Xaa44=C) | SEQ ID NO: 6 |
| CD8(+/-25) | SLRPEACRPAAGGAVHTRGLDFASD (21-45 of SEQ ID NO: 39:Xaa27=C, Xaa44=S) | SEQ ID NO: 6 |
| CD8(-/+25) | SLRPEASRPAAGGAVHTRGLDFACD (21-45 of SEQ ID NO: 39: Xaa27=S,Xaa44=C) | SEQ ID NO: 6 |
| CD8(-/-25) | SLRPEASRPAAGGAVHTRGLDFASD (21-45 of SEQ ID NO: 39: Xaa27=S,Xaa44=S) | SEQ ID NO: 6 |
| CD8(+10) | HTRGLDFACD (36-45 of SEQ ID NO: 39: (Xaa44=C) | SEQ ID NO: 6 |
| C08(-10) | HTRGLDFASD (36-45 of SEQ ID NO: 39: Xaa44=S) | SEQ ID NO: 6 |
| CD28(+39) | IEVMYPPPYLDNEKSNGTIIHVKGKHLCPSPLFPGPSKP (SEQ ID NO: 40: Xaa28=C) | SEQ ID NO: 37 |
| CD28(-39) | IEVMYPPPYLDNEKSNGTIIHVKGKHLSPSPLFPGPSKP (SEQ ID NO: 40: Xaa28=S) | SEQ ID NO: 37 |
| (-) | No linker | SEQ ID NO: 6 |

### [Results]

AChRα-CAAR having each of the aforementioned linker regions/transmembrane domains had significant cytocidal activity which was consistent amongst the donors (Dunnett's-test, p < 0.01). From these results, it was confirmed that in the CAAR of the present invention, the presence or absence of a linker region does not influence activity and the CAAR of the present invention functions as CAAR regardless of the sequence or the length of the linker region.

### <Example 11: In vivo cytocidal activity evaluation of CAAR-T cells>

In order to evaluate *in vivo* cytocidal activity of CAAR-T cells, a pharmacological test was conducted using an immunodeficient, NOG mouse (NOD/Shi-scid, IL-2Rγ<null>). A pathogenic anti-AChRα antibody is present in the body of a myasthenia gravis patient, and the anti-AChRα antibody is presumed to bind to AChRα-CAAR of administered CAAR-T cells, which may in turn undergo elimination from the body through antibody-dependent cytotoxic activity. Despite having low NK cell activity, there is also a possibility that similar elimination occurs in NOG mice due to the presence of macrophages. To control this elimination, treatment with an agent such as intravenous immunoglobulin (IVIg) is probably necessary. Since IVIg is derived from a human, a human-derived constant region of an anti-AChRα antibody (mAb35 antibody) is considered to reflect a real-world clinical situation for the exertion of a suppressive effect thereof. Therefore, in this test, the following experiment was conducted using a humanized mAb35 antibody (mAb35-hIgG1).

mAb35-hIgG1 was obtained by preparing an expression plasmid of a polypeptide (SEQ ID NO: 35) of a mAb35 antibody H chain with its constant region replaced with a human IgG1 constant region and an expression plasmid of a polypeptide (SEQ ID NO: 36) of a mAb35 antibody L chain with its constant region replaced with an Igκ constant region, transfecting HEK293 cells therewith, and purifying a recovered culture supernatant with protein A. A virus vector for expression of AChRα-CAAR (SEQ ID NO: 43) having the amino acid sequence of SEQ ID NO: 48 as a signal peptide and a tag peptide at the N-terminus, and comprising AChRα211/W149R/V155K as an antigenic region was constructed, and CAAR-T cells were prepared.

On the day before the start of the test, IVIg (Baxter, 4987456506071, GAMMAGARD for intravenous injection, 2.5 g) at 10 mg/head and mAb35-hIgG1 at 20 µg/head were administered to the tail vein. The membrane-type mAb35-scFv-expressing RAJI cells prepared in Example 7 were administered at 5 × 10⁵ cells/head to the tail vein. Four hours later, the CAAR-T cells described above were administered at 6 × 10⁶ cells/head to the tail vein. Fourteen days later, the mouse was euthanized, and blood, the spleen, and the bone marrow (the femur and the tibia on one side) were collected and mechanically dispersed.

Each tissue collected was hemolyzed using RBC Lysis buffer (Thermo Fisher Scientific Inc., 00-4333-57). Then, APC anti-DYKDDDDK Tag Antibody (anti-FLAG antibody, BioLegend, Inc., 637308) and PE anti-human CD3 (BioLegend, Inc., 317308) were diluted 100-fold with a FACS buffer, then added to the cells, reacted at 4°C for 15 minutes, and the cells were washed with a FACS buffer. The antibody-stained cells were suspended in a FACS buffer containing 1 µg/mL 7-aminoactinomycin D and measured by use of flow cytometry. After output as an FCS file, a cell fraction was selected in an FSC/SSC plot using FLOW JO software to select viable cells which were negative for 7-aminoactinomycin D staining. Next, the viable cells were gated with ZsGreen1 on the X axis against SSC on the Y axis, and numbers of ZsGreen1-positive cells corresponding to the membrane-type mAb35-scFv-expressing RAJI cells were determined(Figure 13).

### [Results]

Membrane-type mAb35-scFv-expressing RAJI cell counts in blood, the spleen, and the bone marrow were significantly reduced in the AChRα-CAAR-T administration group compared with a control T cell administration group (T-test, p < 0.01).

### <Example 12: Evaluation of cytocidal activity of AChRα-CAAR-T cells against anti-AChRα antibody-producing B cells derived from mouse sensitized with AChRα>

In order to treat myasthenia gravis caused by the production of an anti-AChRα antibody, it is necessary to remove pathogenic B cells leading to autoantibody production. Accordingly, spleen cells including B cells related to anti-AChRα antibody production contained in the spleen of a mouse sensitized with AchRα protein were prepared as target cells that reflected a real-world clinical situation, and the cytocidal activity of AChRα-CAAR-T cells was studied using the amount of an anti-AChRαantibody contained in a culture supernatant as an indicator of cytocidal activity.

### (1) Preparation of CAAR-T cell

Lentivirus vectors enabling the expression of two types of AChRα-CAAR (AChRα/W149R/V155K>4aa>4-1BB: SEQ ID NO: 44, AChRα/W149R/V155K>14aa>OX40: SEQ ID NO: 47) and having the amino acid sequence of SEQ ID NO: 48 as a signal peptide and a tag peptide at the N-terminus. The virus vectors for AChRα-CAAR expression described above were allowed to infect human peripheral blood mononuclear cells to obtain CAAR-T cells.

### (2) Preparation of spleen cell (anti-AChRα antibody-producing B cell)

In order to prepare anti-AChRα antibody-producing cells as target cells, 100 µL of a solution containing 5 to 10 µg of recombinant AChRα protein mixed with Alhydrogel(R) adjuvant 2% (InvivoGen, vac-alu-250) at a ratio of 1:1, was subcutaneously inoculated into a C57BL/6J mouse. Fourteen days later, 5 to 10 µg of recombinant AChRα protein was subcutaneously inoculated thereto as a booster in the same manner as above. After a further 7 days, the mouse was euthanized, and the spleen was then harvested. The spleen was chopped, ground on a 40 µm cell strainer, and suspended in a basal medium to prepare a spleen cell suspension containing anti-AChRα antibody-producing B cells.

### (3) Evaluation of cytocidal activity

A FLAG-positive cell count (CAAR-T cells) was determined using APC anti-DYKDDDDK antibody and flow cytometry for each type of CAAR-T cells prepared as described above. The CAAR-T cells were inoculated into a 96-well plate at a concentration of 1 × 10⁵ cells/well, while the spleen cells were inoculated thereto at a concentration of 1 × 10⁵ cells/well. The medium used was a growth medium supplemented with 0.001% 1-Thioglycerol (Sigma-Aldrich Co., LLC, M6145). A TLR9 ligand K3 (Ajinomoto Bio-Pharma, CN-65003) was added at 1 µM (final concentration) for promoting antibody production. After culture for 8 days, a culture supernatant was recovered. In order to measure the amount of an anti-AChRα antibody in the recovered culture supernatant, AChRα-expressing Jurkat cells were inoculated into a 96-well plate at a concentration of 4 × 10⁴ cells/well, and 40 µL of the culture supernatant was added thereto and reacted at 4°C for 10 to 20 minutes, and the cells were washed with a FACS buffer. APC anti-mouse IgG Antibody (BioLegend, Inc., 405308) was diluted 100-fold with a FACS buffer, then added to the cells, and reacted at 4°C for 10 to 20 minutes, and the cells were washed with a FACS buffer. A FACS buffer containing 2 µg/mL 7-aminoactinomycin D was added in equal amounts thereto, followed by analysis using flow cytometry. After output as an FCS file, a cell fraction was selected in an FSC/SSC plot using FLOW JO software to select live cells which were negative for 7-aminoactinomycin D staining. Next, the viable cells were gated with APC on the X axis against a cell count on the Y axis. MFI was measured to determine the amount of an anti-AChRα antibody contained in the culture supernatant and to indirectly assess the cytocidal activity of the AChRα-CAAR-T cells against the anti-AChRα antibody-producing B cells. The results are shown in Figure 14.

### [Results]

The amount of an anti-AChRα antibody contained in the culture supernatant was reduced in the AChRα-CAAR (4aa/4-1BB and 14aa/OX40)-T cell administration group compared with a control T cell administration group. These results suggested that the AChRα-CAAR-T cells of the present invention can target and kill B cells producing an anti-AChRα antibody.

### Free Text of Sequence Listing

SEQ ID NO: 1: AChRα236 (human native AChR α subunit extracellular region)
SEQ ID NO: 2: AChRα211 (Δ59-74 human AChR α subunit extracellular region)
SEQ ID NO: 3: AChRα211 mutant (Xaa: 8, 14, 70, 72, 112 146-159, 192, 193)
SEQ ID NO: 4: AChRα236 mutant (Xaa: 8, 171-184)
SEQ ID NO: 5: CD8a linker region
SEQ ID NO: 6: CD8a transmembrane domain
SEQ ID NO: 7: 4-1BB co-stimulatory domain
SEQ ID NO: 8: CD3ζ intracellular signal domain
SEQ ID NO: 9: CD8α signal peptide
SEQ ID NO: 10: human AChRα signal peptide
SEQ ID NO: 11: human immunoglobulin signal peptide
SEQ ID NO: 12: AChRβ
SEQ ID NO: 13: AChRδ
SEQ ID NO: 14: AChRε
SEQ ID NO: 15: main immunogenic region
SEQ ID NO: 16: hydrophobic region (146-159), G147K mutation
SEQ ID NO: 17: hydrophobic region (146-159), W149K mutation
SEQ ID NO: 18: hydrophobic region (146-159), Y151K mutation
SEQ ID NO: 19: hydrophobic region (146-159), G153K mutation
SEQ ID NO: 20: hydrophobic region (146-159), V155K mutation
SEQ ID NO: 21: hydrophobic region (146-159), A157K mutation
SEQ ID NO: 22: hydrophobic region (146-159), N159K mutation
SEQ ID NO: 23: hydrophobic region (146-159), V155K mutation
SEQ ID NO: 24: membrane-type mAb35-scFv
SEQ ID NO: 25: CD28 co-stimulatory domain
SEQ ID NO: 26: GITR co-stimulatory domain
SEQ ID NO: 27: TNFR2 co-stimulatory domain
SEQ ID NO: 28: DR3 co-stimulatory domain
SEQ ID NO: 29: CD30 co-stimulatory domain
SEQ ID NO: 30: HVEM co-stimulatory domain
SEQ ID NO: 31: CD27 co-stimulatory domain
SEQ ID NO: 32: OX40 co-stimulatory domain
SEQ ID NO: 33: humanized membrane-type mAb35
SEQ ID NO: 34: CD79a/CD79b
SEQ ID NO: 35: mAb35-hIgG1
SEQ ID NO: 36: mAb35-hIgGκ
SEQ ID NO: 37: CD28 transmembrane domain
SEQ ID NO: 38: AChR transmembrane domain
SEQ ID NO: 39: CD8α linker region (Xaa27,44 = C or S)
SEQ ID NO: 40: CD28 linker region (Xaa28 = C or S)
SEQ ID NO: 42: AChRα/W149X/V155X
SEQ ID NO: 43 to 47: AChRα-CAAR full-length sequence
SEQ ID NO: 48: signal-FLAG-peptide

## Claims

1. A chimeric polypeptide receptor in which an extracellular region comprising an antigenic region capable of being bound by an anti-human nicotinic acetylcholine receptor α1 subunit (nAChRα1) antibody, a transmembrane region, and an intracellular region comprising an intracellular signaling domain are arranged in the presented order from the N-terminus towards the C-terminus, wherein the antigenic region comprises the amino acid sequence as set forth in SEQ ID NO: 2 or an amino acid sequence derived from the amino acid sequence as set forth in SEQ ID NO: 2 by the substitution, deletion, insertion, and/or addition of one or several amino acids.

2. The polypeptide receptor according to claim 1, wherein the antigenic region is a region consisting of an amino acid sequence derived from the amino acid sequence as set forth in SEQ ID NO: 2 by the substitution of at least one amino acid represented by Xaa in SEQ ID NO: 3 and optionally by the deletion, insertion, and/or addition of one or several amino acids at positions other than amino acids represented by Xaa in SEQ ID NO: 3 as one or more additional mutations.

3. The polypeptide receptor according to claim 2, wherein the one or more additional mutations is the deletion or addition of one to five N-terminal and/or C-terminal amino acids in SEQ ID NO: 2.

4. The polypeptide according to claim 2 or 3, wherein in the amino acid sequence of SEQ ID NO: 3, the amino acid represented by Xaa is as follows:
Xaa8 is a hydrophobic amino acid or an acidic amino acid,
Xaa14 is an acidic amino acid or a hydrophobic amino acid,
Xaa70 is an acidic amino acid, an amino acid having an a side chain comprising an amide group, or a hydrophobic amino acid,
Xaa72 is a hydrophobic amino acid,
Xaa112 is a hydrophobic amino acid,
Xaa149 is a hydrophilic amino acid or a hydrophobic amino acid,
Xaa155 is a hydrophobic amino acid or a hydrophilic amino acid,
amino acids Xaa146 to Xaa148, Xaa150 to Xaa154, and
Xaa156 to Xaa159 are each independently a basic amino acid or a hydrophobic amino acid, and
Xaa192 and Xaa193 are each independently Cys or Gly,
wherein the hydrophobic amino acid is Val, Ala, Leu, Ile, Gly, Trp, Tyr, Phe, Met, or Pro, the hydrophilic amino acid is Arg, Lys, Asp, Glu, Asn, Gln, or Ser, the acidic amino acid is Asp or Glu, the basic amino acid is Arg or
Lys, and the amino acid having a side chain comprising an amide group is Asn or Gln.

5. The polypeptide receptor according to claim 4, wherein in the amino acid sequence of SEQ ID NO: 3, the amino acids represented by Xaa is as follows:
Xaa8 is Val or Glu,
Xaa14 is Asp, Ala, or Gly,
Xaa70 is Asp, Asn, or Ala,
Xaa72 is Tyr or Phe,
Xaa112 is Tyr or Phe,
Xaa149 is Trp, Arg, Ala, Lys, Asp, Ser, Gly, Asn, Ile, Phe, Met, or Pro,
Xaa155 is Trp, Arg, Ala, Lys, Asp, Ser, Gly, Asn, Ile, Phe, Met, or Pro,
amino acids Xaa146 to Xaa148, Xaa150 to Xaa154, and
Xaa156 to Xaa159 are each independently the amino acid of the corresponding site in SEQ ID NO: 2 or Lys, and
Xaa192 and Xaa193 are each independently Cys or Gly.

6. The polypeptide receptor according to claim 5,
wherein in the amino acid sequence of SEQ ID NO: 3,
Xaa149 is Trp, Lys, Arg, or Pro, and Xaa155 is Val, Ala, Gly, Lys, Arg, Pro, Met, Asp, Asn, Glu, Gln, or Ser.

7. The polypeptide receptor according to claim 6, wherein in the amino acid sequence of SEQ ID NO: 3, Xaa149 and Xaa155 are each independently Lys or Arg.

8. The polypeptide receptor according to claim 1, wherein the antigenic region is any one member selected from the following group:
antigenic region: AChRα211, AChRα211/V8E, AChRα211/W149R,
AChRα211/W149K, AChRα211/W149P, AChRα211/V155A,
AChRα211/V155K, AChRα211/V155M, AChRα211/V155D,
AChRα211/V155P, AChRα211/C192G, AChRα211/C193G,
AChRα211/C192G/C193G, AChRα211/V8E/W149R,
AChRα211/V8E/W149K, AChRα211/V8E/W149P,
AChRα211/V8E/V155A, AChRα211/V8E/V155K,
AChRα211/V8E/V155M, AChRα211/V8E/V155D,
AChRα211/V8E/V155P, AChRα211/V8E/C192G,
AChRα211/V8E/C193G, AChRα211/V8E/C192G/C193G,
AChRα211/W149R/V155A, AChRα211/W149R/V155K,
AChRα211/W149R/V155M, AChRα211/W149R/V155D,
AChRα211/W149R/V155P, AChRα211/W149R/C192G,
AChRα211/W149R/C193G, AChRα211/W149R/C192G/C193G,
AChRα211/W149K/V155A, AChRα211/W149K/V155K,
AChRα211/W149K/V155M, AChRα211/W149K/V155D,
AChRα211/W149K/V155P, AChRα211/W149K/C192G,
AChRα211/W149K/C193G, AChRα211/W149K/C192G/C193G,
AChRα211/W149P/V155A, AChRα211/W149P/V155K,
AChRα211/W149P/V155M, AChRα211/W149P/V155D,
AChRα211/W149P/V155P, AChRα211/W149P/C192G,
AChRα211/W149P/C193G, AChRα211/W149P/C192G/C193G,
AChRα211/V8E/W149R/V155A, AChRα211/V8E/W149R/V155K,
AChRα211/V8E/W149R/V155M, AChRα211/V8E/W149R/V155D,
AChRα211/V8E/W149R/V155P, AChRα211/V8E/W149R/C192G,
AChRα211/V8E/W149R/C193G, AChRα211/V8E/W149R/C192G/C193G,
AChRα211/V8E/W149K/V155A, AChRα211/V8E/W149K/V155K,
AChRα211/V8E/W149K/V155M, AChRα211/V8E/W149K/V155D,
AChRα211/V8E/W149K/V155P, AChRα211/V8E/W149K/C192G,
AChRα211/V8E/W149K/C193G, AChRα211/V8E/W149K/C192G/C193G,
AChRα211/V8E/W149P/V155A, AChRα211/V8E/W149P/V155K,
AChRα211/V8E/W149P/V155M, AChRα211/V8E/W149P/V155D,
AChRα211/V8E/W149P/V155P, AChRα211/V8E/W149P/C192G,
AChRα211/V8E/W149P/C193G, AChRα211/V8E/W149P/C192G/C193G,
AChRα211/W149R/V155A/C192G, AChRα211/W149R/V155K/C192G,
AChRα211/W149R/V155M/C192G, AChRα211/W149R/V155D/C192G,
AChRα211/W149R/V155P/C192G, AChRα211/W149K/V155A/C192G,
AChRα211/W149K/V155K/C192G, AChRα211/W149K/V155M, /C192G,
AChRα211/W149K/V155D/C192G, AChRα211/W149K/V155P/C192G,
AChRα211/W149P/V155A/C192G, AChRα211/W149P/V155K/C192G,
AChRα211/W149P/V155M, /C192G, AChRα211/W149P/V155D/C192G,
AChRα211/W149P/V155P/C192G, AChRα211/W149R/V155A/C193G,
AChRα211/W149R/V155K/C193G, AChRα211/W149R/V155M/C193G,
AChRα211/W149R/V155D/C193G, AChRα211/W149R/V155P/C193G,
AChRα211/W149K/V155A/C193G, AChRα211/W149K/V155K/C193G,
AChRα211/W149K/V155M/C193G, AChRα211/W149K/V155D/C193G,
AChRα211/W149K/V155P/C193G, AChRα211/W149P/V155A/C193G,
AChRα211/W149P/V155K/C193G, AChRα211/W149P/V155M/C193G,
AChRα211/W149P/V155D/C193G, AChRα211/W149P/V155P/C193G,
AChRα211/W149R/V155A/C192G/C193G,
AChRα211/W149R/V155K/C192G/C193G,
AChRα211/W149R/V155M/C192G/C193G,
AChRα211/W149R/V155D/C192G/C193G,
AChRα211/W149R/V155P/C192G/C193G,
AChRα211/W149K/V155A/C192G/C193G,
AChRα211/W149K/V155K/C192G/C193G,
AChRα211/W149K/V155M/C192G/C193G,
AChRα211/W149K/V155D/C192G/C193G,
AChRα211/W149K/V155P/C192G/C193G,
AChRα211/W149P/V155A/C192G/C193G,
AChRα211/W149P/V155K/C192G/C193G,
AChRα211/W149P/V155M/C192G/C193G,
AChRα211/W149P/V155D/C192G/C193G,
AChRα211/W149P/V155P/C192G/C193G,
AChRα211/D14G/W149R/V155K, AChRα211/D14G/W149R/V155A,
AChRα211/D14G/W149R/V155M, AChRα211/D14G/W149R/V155D,
AChRα211/D14G/W149R/V155P, AChRα211/D14G/W149R/V155G,
AChRα211/D14G/W149K/V155K, AChRα211/D14G/W149K/V155A,
AChRα211/D14G/W149K/V155M, AChRα211/D14G/W149K/V155D,
AChRα211/D14G/W149K/V155P, AChRα211/D14G/W149K/V155G,
AChRα211/D14G/W149P/V155K, AChRα211/D14G/W149P/V155A,
AChRα211/D14G/W149P/V155M, AChRα211/D14G/W149P/V155D,
AChRα211/D14G/W149P/V155P, AChRα211/D14G/W149P/V155G,
AChRα211/D14A/W149R/V155K, AChRα211/D14A/W149R/V155A,
AChRα211/D14A/W149R/V155M, AChRα211/D14A/W149R/V155D,
AChRα211/D14A/W149R/V155P, AChRα211/D14A/W149R/V155G,
AChRα211/D14A/W149K/V155K, AChRα211/D14A/W149K/V155A,
AChRα211/D14A/W149K/V155M, AChRα211/D14A/W149K/V155D,
AChRα211/D14A/W149K/V155P, AChRα211/D14A/W149K/V155G,
AChRα211/D14A/W149P/V155K, AChRα211/D14A/W149P/V155A,
AChRα211/D14A/W149P/V155M, AChRα211/D14A/W149P/V155D,
AChRα211/D14A/W149P/V155P, or, AChRα211/D14A/W149P/V155G
wherein AChRα211 is the amino acid sequence as set forth in SEQ ID NO: 2, the position of each of the mentioned substitutions of AChRα211 refers to the corresponding position in the amino acid sequence represented by SEQ ID NO: 2, and in each substitution the substituting amino acid is indicated after the position.

9. The polypeptide receptor according to any one of claims 1 to 8, wherein the intracellular signaling domain is an amino acid sequence derived from an intracellular domain of CD3ζ or CD3δ.

10. The polypeptide receptor according to any one of claims 1 to 8, wherein the transmembrane region is an amino acid sequence derived from a transmembrane domain of any one molecule selected from nAChRα1, T cell receptor α or β chain, CD3ζ chain, CD28, CD3ε, CD45, CD4, CD5, CD8, CD9, CD16, CD22, CD33, CD37, CD64, CD80, CD86, CD134, CD137, ICOS, CD154, and GITR.

11. The polypeptide receptor according to any one of claims 1 to 10, wherein the intracellular region further comprises one to three co-stimulatory domains on the N-terminus of the intracellular signaling domain.

12. The polypeptide receptor according to claim 11, wherein the co-stimulatory domains are sequences derived from intracellular regions of one to three molecules selected from the group consisting of CD2, CD4, CD5, CD8α, CD8β, CD28, CD134, CD137 (4-1BB), ICOS, CD154, CITR, TNFR2, DR3, CD30, HVEM, CD27, and OX40.

13. The polypeptide receptor according to any one of claims 1 to 12, further comprising a linker region consisting of 100 or less amino acids between the antigenic region and the transmembrane region.

14. The polypeptide receptor according to claim 13, wherein the linker region is a portion or the whole of the amino acid sequence as set forth in SEQ ID NO: 5, 39, 40, or 41.

15. The polypeptide according to claim 1, wherein
the antigenic region is AChRα211/W149R/V155K,
AChRα211/W149R/V155A, AChRα211/W149R/V155M,
AChRα211/W149R/V155D, AChRα211/W149R/V155P,
AChRα211/W149R/V155G, AChRα211/W149K/V155K,
AChRα211/W149K/V155A, AChRα211/W149K/V155M,
AChRα211/W149K/V155D, AChRα211/W149K/V155P,
AChRα211/W149K/V155G, AChRα211/W149P/V155K,
AChRα211/W149P/V155A, AChRα211/W149P/V155M,
AChRα211/W149P/V155D, AChRα211/W149P/V155P,
AChRα211/W149P/V155G, AChRα211/D14G/W149R/V155K,
AChRα211/D14G/W149R/V155A, AChRα211/D14G/W149R/V155M,
AChRα211/D14G/W149R/V155D, AChRα211/D14G/W149R/V155P,
AChRα211/D14G/W149R/V155G, AChRα211/D14G/W149K/V155K,
AChRα211/D14G/W149K/V155A, AChRα211/D14G/W149K/V155M,
AChRα211/D14G/W149K/V155D, AChRα211/D14G/W149K/V155P,
AChRα211/D14G/W149K/V155G, AChRα211/D14G/W149P/V155K,
AChRα211/D14G/W149P/V155A, AChRα211/D14G/W149P/V155M,
AChRα211/D14G/W149P/V155D, AChRα211/D14G/W149P/V155P,
AChRα211/D14G/W149P/V155G, AChRα211/D14A/W149R/V155K,
AChRα211/D14A/W149R/V155A, AChRα211/D14A/W149R/V155M,
AChRα211/D14A/W149R/V155D, AChRα211/D14A/W149R/V155P,
AChRα211/D14A/W149R/V155G, AChRα211/D14A/W149K/V155K,
AChRα211/D14A/W149K/V155A, AChRα211/D14A/W149K/V155M,
AChRα211/D14A/W149K/V155D, AChRα211/D14A/W149K/V155P,
AChRα211/D14A/W149K/V155G, AChRα211/D14A/W149P/V155K,
AChRα211/D14A/W149P/V155A, AChRα211/D14A/W149P/V155M,
AChRα211/D14A/W149P/V155D, AChRα211/D14A/W149P/V155P, or
AChRα211/D14A/W149P/V155G,
wherein AChRα211 is the amino acid sequence as set forth in SEQ ID NO: 2, the position of each of the mentioned substitutions of AChRα211 refers to the corresponding position in the amino acid sequence represented by SEQ ID NO: 2, and in each substitution, the substituting amino acid is indicated after the position,
the linker region is a portion or the whole of a CD8α hinge sequence (SEQ ID NO: 39), a CD28 hinge sequence (SEQ ID NO: 40), or an artificial linker sequence (SEQ ID NO: 41),
the transmembrane region is a CD8α transmembrane domain (SEQ ID NO: 6), a CD28 transmembrane domain (SEQ ID NO: 37), or an AChRα transmembrane domain (SEQ ID NO: 38),
the co-stimulatory domain is a 4-1BB co-stimulatory domain (SEQ ID NO: 7), a CD28 co-stimulatory domain (SEQ ID NO: 25), a GITR co-stimulatory domain (SEQ ID NO: 26), a TNFR2 co-stimulatory domain (SEQ ID NO: 27), a DR3 co-stimulatory domain (SEQ ID NO: 28), a CD30 co-stimulatory domain (SEQ ID NO: 29), an HVEM co-stimulatory domain (SEQ ID NO: 30), a CD27 co-stimulatory domain (SEQ ID NO: 31), or an OX40 co-stimulatory domain (SEQ ID NO: 32), and
the intracellular signaling domain is a CD3ζ intracellular signaling domain (SEQ ID NO: 8).

16. The polypeptide receptor according to claim 15, wherein
the antigenic region is AChRα211/W149R/V155K (which is an amino acid sequence of SEQ ID NO: 42 wherein Xaa14 is Asp, Xaa149 is Arg, and Xaa155 is Lys), AChRα211/W149R/V155A (which is an amino acid sequence of SEQ ID NO: 42 wherein Xaa14 is Asp, Xaa149 is Arg, and Xaa155 is Ala), AChRα211/D14G/W149R/V155K (which is an amino acid sequence of SEQ ID NO: 42 wherein Xaa14 is Gly, Xaa149 is Arg, and Xaa155 is Lys), or AChRα211/ D14G/W149R/V155A (which is an amino acid sequence of SEQ ID NO: 42 wherein Xaa14 is Gly, Xaa149 is Arg, and Xaa155 is Ala),
the linker region is a CD8α hinge sequence (SEQ ID NO: 39), 14 consecutive amino acids from the C-terminus thereof (which are an amino acid sequence consisting of amino acids at positions 32 to 45 of SEQ ID NO: 39), an artificial linker sequence (SEQ ID NO: 41), or 4 consecutive amino acids from the C-terminus thereof (which are an amino acid sequence consisting of amino acids at positions 11 to 14 of SEQ ID NO: 41),
the transmembrane region is a CD8α transmembrane domain (SEQ ID NO: 6),
the co-stimulatory domain is a 4-1BB co-stimulatory domain (SEQ ID NO: 7) or an OX40 co-stimulatory domain (SEQ ID NO: 32), and
the intracellular signaling domain is a CD3ζ intracellular signaling domain (SEQ ID NO: 8).

17. A polypeptide consisting of an amino acid sequence, wherein the amino acid sequence of the polypeptide comprises an amino acid sequence of a signal peptide for enabling expression of the polypeptide on the cell surface, and further comprises an amino acid sequence of a chimeric receptor that exhibits 800 or higher sequence identity to an amino acid sequence of any of SEQ ID NOs: 43 to 47.

18. The polypeptide according to claim 17, wherein the amino acid sequence of the chimeric receptor consists of an amino acid sequence of any of SEQ ID NOs: 43 to 47.

19. A polynucleotide encoding a polypeptide according to any one of claims 1 to 18.

20. A vector comprising a polynucleotide according to claim 19.

21. The vector according to claim 20, wherein the vector is any vector selected from a plasmid vector, a retrovirus vector, and a lentivirus vector.

22. The vector according to claim 20 or 21, wherein the vector is designed to enable expression of a polypeptide according to any one of claims 1 to 18.

23. The vector according to claim 20 or 21, wherein the vector is a plasmid vector designed to enable production of a virus vector in which a polynucleotide encoding a polypeptide according to any one of claims 1 to 18 is enclosed.

24. A cell transfected with a polynucleotide according to claim 19.

25. The cell according to claim 24, wherein the cell expresses the polypeptide according to any of claims 1 to 18 on the cell surface.

26. The cell according to claim 24 or 25, wherein the cell is any member selected from a T cell, PBMC, an iPS cell, and an ES cell.

27. A medicament for treating a disease associated with the production of an autoantibody which binds to an acetylcholine receptor α subunit, the medicament comprising a cell according to any one of claims 24 to 26 as an active ingredient.

28. The medicament according to claim 27, wherein the disease is myasthenia gravis.

29. A method for treating myasthenia gravis, comprising a step of administering a therapeutically effective amount of a cell according to any one of claims 24 to 26 to a subject to be treated.

30. The cell according to any one of claims 24 to 26 for use in the treatment of myasthenia gravis.

31. Use of a cell according to any one of claims 24 to 26 for the production of a drug for treating myasthenia gravis.
